(19) Europäisches Patentamt
European Patent Office
Office européen des brevets

(11) **EP 2 271 419 B1**

(12) **EUROPÄISCHE PATENTSCHRIFT**

(45) Veröffentlichungstag und Bekanntmachung des Hinweises auf die Patenterteilung:
**18.10.2017 Patentblatt 2017/42**

(51) Int Cl.:
*B01D 69/02* (2006.01)    *B01D 71/10* (2006.01)
*B01D 67/00* (2006.01)    *B01J 20/28* (2006.01)
*B01D 15/34* (2006.01)    *C07K 1/22* (2006.01)
*C07K 1/36* (2006.01)    *C08B 1/08* (2006.01)
*C08B 15/10* (2006.01)

(21) Anmeldenummer: **09733075.7**

(22) Anmeldetag: **10.02.2009**

(86) Internationale Anmeldenummer:
**PCT/EP2009/000915**

(87) Internationale Veröffentlichungsnummer:
**WO 2009/127287 (22.10.2009 Gazette 2009/43)**

(54) **VERFAHREN ZUR STOFFTRENNUNG UNTER VERWENDUNG EINER CELLULOSEHYDRAT-MEMBRAN IN DER SIZE EXCLUSION CHROMATOGRAPHY**

METHOD FOR SUBSTANCE SEPARATION USING A CELLULOSE HYDRATE MEMBRANE IN SIZE EXCLUSION CHROMATOGRAPHY

PROCÉDÉ DE SÉPARATION DE SUBSTANCES UTILISANT UNE MEMBRANE D'HYDRATE DE CELLULOSE DANS LA CHROMATOGRAPHIE D'EXCLUSION STÉRIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **14.04.2008 DE 102008018732**

(43) Veröffentlichungstag der Anmeldung:
**12.01.2011 Patentblatt 2011/02**

(73) Patentinhaber: **Sartorius Stedim Biotech GmbH 37079 Göttingen (DE)**

(72) Erfinder:
• **FABER, René**
**37077 Göttingen (DE)**

• **DEMMER, Wolfgang**
**37077 Göttingen (DE)**

(74) Vertreter: **Müller-Boré & Partner Patentanwälte PartG mbB Friedenheimer Brücke 21 80639 München (DE)**

(56) Entgegenhaltungen:
WO-A-03/015902    WO-A-2007/017085
WO-A-2008/095709    DE-A1- 4 323 913
US-A- 6 103 121    US-A1- 2003 038 081

**Beschreibung**

Die vorliegende Erfindung betrifft ein Verfahren für die Stofftrennung in flüssigen Medien aufgrund einer Kombination von adsorptiver Wechselwirkung des Adsorbenden mit dem Adsorbens und des Größenausschlußeffektes. Insbesondere betrifft die vorliegende Erfindung ein Verfahren, das eine Kombination von Membranchromatographie und Größenausschlusschromatographie für die Stofftrennung nutzt.

Physikalische Trennverfahren, bei denen die Stofftrennung durch Verteilung zwischen einer stationären und einer mobilen Phase geschieht, kennt man unter dem Begriff Chromatographie. Das Gleichgewicht kann sich aufgrund verschiedener physikalisch-chemischer Effekte ausbilden.

Erfolgt die Verteilung durch Adsorption an einem Feststoff (Adsorbens) als stationärer Phase, spricht man von Adsorptions-Chromatographie, z.B. Ionenaustauschchromatographie, Affinitätschromatographie und hydrophobe Interaktionschromatographie.

Bei der Gel-Permeations-Chromatographie (GPC) handelt es sich um eine Art der Flüssigchromatographie. Die Trennung findet hier jedoch ausschließlich aufgrund der Größe (genauer: des hydrodynamischen Volumens) der Moleküle in Lösung statt. Weitere Bezeichnungen sind Größenausschlußchromatographie oder, englisch, Size Exclusion Chromatography (SEC). Wird mit einem wässrigen Laufmittel gearbeitet, so verwendet man auch häufig den Begriff der Gelfiltration oder Gelfiltrations-Chromatographie (GFC).

Unter Filtration versteht man ein Verfahren zur Trennung von Feststoffteilchen oder Molekülen aus Flüssigkeiten (z.B. Suspensionen, Proteinlösungen) oder aus Gasen (z.B. Staub), auch von nichtlöslichen Flüssigkeitströpfchen aus einer anderen Flüssigkeit (Emulsion) oder aus Gasen (Aerosolen). Gemeinsames wesentliches Merkmal der Filtration ist, daß ein poröses Medium (z.B. Filterpapier, Membran) von der kontinuierlichen Phase (Flüssigkeit oder Gas) durchströmt wird, wobei gleichzeitig die Feststoffteilchen, Moleküle oder Tröpfchen an der Oberfläche des porösen Mediums oder in seinem Inneren zurückgehalten werden (Retention).

Poröse Membranen werden hauptsächlich in den Verfahren der Ultrafiltration, der Mikrofiltration und der Dialyse eingesetzt. Ob ein Partikel oder Molekül von Ultra- oder Mikrofiltrationsmembranen zurückgehalten wird, hängt neben den Betriebsbedingungen vor allem von seiner Größe und Struktur relativ zur Größe und Struktur der Membranporen ab. Typischer Einsatzbereich der Mikrofiltration ist z.B. die Konzentrierung von Suspensionen, wobei die Ultrafiltration oft zur Fraktionierung von niedermolekularen gelösten Stoffen und Makromolekülen eingesetzt wird. Eine vollständige Trennung bei der Ultrafiltration erfordert in diesem Zusammenhang, daß die Molkulargewichte der zu fraktionierenden Stoffe sich mindestens um eine Größenordnung unterscheiden.

Die Porengröße von Mikrofiltrationsmembranen liegt im Mikrometerbereich (etwa 0,08 bis etwa 10 $\mu$m). Die Porengröße der Ultrafiltrationsmembranen wird meist mit der Angabe der Grenze, bei der 90 % (oder 95 %) der Moleküle einer bestimmten Molmasse zurückgehalten werden (molekulare Trenngrenze, molecular weight cutoff, MWCO), definiert.

Unter Selektivität einer Membarn versteht man ihre Fähigkeit, zwischen den Komponenten einer Mischung zu unterscheiden.

Adsorptive Membranen, auch Membranadsorber genannt, sind im Stand der Technik bekannt. Zum Hervorrufen der rein adsorptiven Trennung werden meistens Mikrofiltrationsmembranen mit funktionellen Gruppen (Liganden) modifiziert. Die Ausführung chromatographischer Trennungen mit Hilfe von Adsorptionsmembranen wird auch Membranchromatographie genannt und sämtliche der in der Chromatographie bekannten synthetischen und natürlichen Liganden können in gleicher Weise auch für Adsorptionsmembranen eingesetzt werden. Das flächige Adsorbens besteht meist aus einer oder mehreren Lagen einer adsorptiven Membran. Da Filtrationseffekte mit den adsorptiven Membranen eher unerwünscht sind, liegen die Porengrößen der im Industriemaßstab verwendeten adsorptiven Membranen meistens im Bereich von >0,4 $\mu$m. Aufgrund dieser Porengröße zeigen solche Membranen meistens nur eine schwache Abhängigkeit der Proteinbindung von der Proteingröße.

Die Bindung der Adsorbenden an das Adsorbens kann reversibel oder irreversibel sein, in jedem Fall ermöglicht sie ihre Abtrennung von den Fluiden, bei denen es sich im allgemeinen um wässrige Flüssigkeiten handelt und die im folgenden Medien genannt werden. Unter dem Begriff "Elution" werden die Desorption und die damit einhergehenden Spülschritte zusammengefasst und die zur Elution verwendete Flüssigkeit ist das "Eluens". Die Komponenten können eine oder mehrere Zielsubstanzen und/oder eine oder mehrere Kontaminanten darstellen. "Zielsubstanzen" sind Wertstoffe, die aus dem Medium in angereicherter oder reiner Form gewonnen werden sollen. "Kontaminanten" sind Stoffe, deren Abwesenheit aus technischen, regulatorischen oder sonstigen Gründen erforderlich oder wünschenswert ist. Für die Entfernung von Kontaminanten, die als "negative Adsorption" bezeichnet wird, kann (darf) die Adsorption irreversibel verlaufen, wenn das Adsorbens nur einmal verwendet werden soll. Bei der Adsorption der Zielsubstanz(en) muß der Vorgang reversibel verlaufen. Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können.

Geladene Ultrafiltrationsmembranen und das Verfahren "high performance tangential flow filtration" (HPTFF) sind in dem Stand der Technik bekannt (beispielsweise aus dem Patent US 7,153, 426 B2)). Die Ladung an der Membranoberfläche sollte die retentive Filtrationsselektivität der Ultrafiltrationsmembran verbessern.

Der Artikel "Alkaline treatment of the cellulose fiber affecting membrane column behaviour for high-performance immunoaffinity chromatography", von Dongmei Zhou, Hanfa Zou, Hailin Wang, Jianyi Ni, Qiang Zhang und Yukui Zhang in Biomed. Chromatogr. 14 (2000), 511-515, beschreibt Eigenschaften von laugebehandelten Cellulosefasern, die mit Acrylaten und anschließend Protein A modifiziert wurden. Die Fasern zeigen Unterschiede in den Zugänglichkeiten für Proteine, jedoch nur geringe Unterschiede zwischen der laugebehandelten und der unbehandelten Fasern.

WO 03/015902 A2 betrifft eine Cellulosemembran, wobei die Membran aus einem Material gegossen worden ist, das ein Cellulosepolymer und ein Lösungsmittel umfasst, wobei die Membran eine erste poröse Fläche mit einem ersten durchschnittlichen Porendurchmesser, eine zweite poröse Fläche mit einem zweiten durchschnittlichen Porendurchmesser, und eine poröse Trägerstruktur dazwischen aufweist, wobei die Trägerstruktur ein netzförmiges Netzwerk aus Strömungskanälen umfasst, wobei der erste und der zweite durchschnittliche Porendurchmesser eine Asymmetrie von mindestens etwa 2:1 aufweisen, und wobei die porösen Flächen und die poröse Trägerstruktur ein Netzwerk von strukturellen Oberflächen aufweisen, die einen Filterstrom kontaktieren können.

US 6,103,121 betrifft ein Verfahren zur Herstellung eines Sorptionsmittels auf Membranbasis, das für eine Metallionenkomplexierung geeignet ist, umfassend das selektive Hydrolysieren einer mikroporösen Membran, die aus einem polyacetylierten Cellulosematerial aufgebaut ist, um eine Oberflächenschicht der Membran zu deacetylieren und freie Hydroxylgruppen freizulegen, das Oxidieren der freigelegten Hydroxylgruppen zu Aldehydgruppen und das Binden einer Polyaminosäure an die Cellulosemembran mittels der Aldehydgruppen.

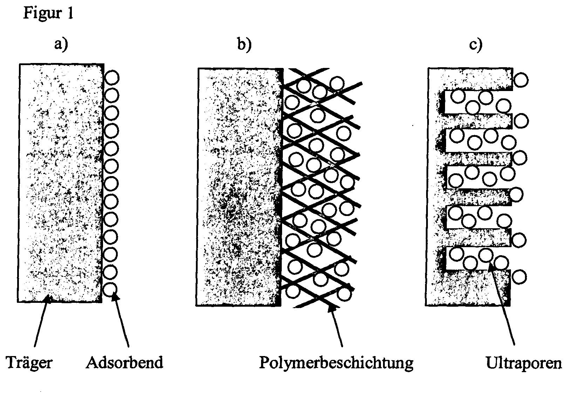

In WO 2007/017085 A2 wird ein Verfahren zur Herstellung von vernetzten Cellulosehydrat-Membranen beschrieben, das in der simultanen Verseifung und Vernetzung von Celluloseester-Membranen besteht und für Filtrations- und Adsorptionsmembranen gleichermaßen geeignet sein soll. Eines der Ziele der dort beschriebenen Erfindung ist die Verseifung und Vernetzung des Celluloseesters unter Bedingungen, die die Struktur und Permeabilität der Membran nicht beeinflussen. Da sich die Struktur der Membran im simultan ablaufenden Verseifungs- und Vernetzungsprozess nicht ändert, ist davon auszugehen, daß die Adsorption von Adsorbenden an der Oberfläche der Mikroporen des Trägers, die der Oberfläche der Ausgangs-Celluloseester-Membran entspricht, stattfindet (vgl. fig. 1a).

DE 43 23 913 A1 beschreibt Trennmaterialien für die hydrophobe Chromatographie auf der Grundlage von hydroxylgruppenhaltigen Basisträgern, auf deren Oberflächen Polymere kovalent gebunden sind.

WO 2008/095709 A1, welche Stand der Technik gemäß Art. 54(3) EPÜ darstellt, beschreibt ein Verfahren zur Herstellung einer porösen, vernetzten, geladenen Cellulosepolymermembran, wobei die Cellulosepolymermembran in Gegenwart einer Base vernetzt wird und anschließend geladene Liganden in diese Membran eingeführt werden, und die so erhaltene Membran in Wasser eine Quellung von 40 bis 250 Vol.-% aufweist.

In der vorliegenden Erfindung werden folgende Abkürzungen verwendet:

| | |
|---|---|
| CA | Celluloseacetat |
| FPLC | Fast Protein Liquid Chromatography |
| Glob | $\gamma$-Globulin |
| GPC | Gel Permeation Chromatography |
| HPLC | High Pressure Liquid Chromatography |
| IP | Isoelektrischer Punkt |
| Kap | Kapazität für Proteine |
| kDa | Kilodalton |
| KPi | Kaliumphosphatpuffer |
| LF | Leitfähigkeit |
| Lys | Lysozym |
| MG | Molekulargewicht |
| NaCl | Natriumchlorid |
| NaPi | Natriumphosphat |
| NaOH | Natriumhydroxid |
| $Na_2HPO_4$ | Di-Natriumhydrogenphosphat |
| S | Sulfonsäure |
| SEC | Size Exclusion Chromatography |
| UV | Ultraviolett |

Der vorliegenden Erfindung liegt die Aufgabe zugrunde, ein Verfahren bereitzustellen, das eine Trennung von Molekülen sowohl aufgrund ihrer unterschiedlichen adsorptiven Eigenschaften als auch aufgrund ihrer Größen ermöglicht.

Diese Aufgaben werden durch die in den Patentansprüchen gekennzeichneten Gegenstände gelöst.

Gegenstand der Erfindung ist somit ein Verfahren zur Trennung von Molekülen durch Kombination von Membranchromatographie und Größenausschlußchromatographie.

Das erfindungsgemäße Trennverfahren nutzt somit die Kombination aus Adsorption von Adsorbenden an der Membranoberfläche und Größenausschlußeffekte. So wird die Selektivität der adsorptiven Membran verbessert, was zur Verbesserung von Aufarbeitungsprozessen führen kann.

Das erfindungsgemäße Trennverfahren kann beispielsweise bei speziellen Trennaufgaben dann vorteilhaft sein, wenn die Spezifität des Liganden eines Membranadsorbers für die Stofftrennung alleine nicht ausreicht und die Molmassen der zu trennenden Komponenten so stark unterschiedlich sind, daß sich aus der Überlagerung der rein adsorptiven Stofftrennung mit einem Größenausschlußeffekt insgesamt eine Verbesserung der Trennleistung ergibt. Eine vollständige Trennung auf der Basis allein dieses Größenausschlußeffekts ist allerdings nicht möglich, weil der Größenausschluß nur für die Adsorption an der inneren Oberfläche der (wie nachstehend definierten) Ultraporen wirksam wird, nicht aber an der äußeren Oberfläche der (wie nachstehend definierten) Mikroporen. Das erfindungsgemäße Verfahren erlaubt z.B. die Trennung von zwei gleich geladenen Proteinen, die sich in ihrer Größe unterscheiden (z.B. Lysozym und $\gamma$-Globulin), auf einer Kationenaustauschermembran.

Gemäß der vorliegenden Erfindung können die Mikroporen, die sich von der ersten Hauptoberfläche der Membran durch die Membran zur zweiten Hauptoberfläche erstrecken, unter Ausbildung von Kanälen kommunizierend miteinander verbunden sein und die Ultraporen können sich sackförmig von der inneren Oberfläche der Mikroporen in das die Struktur der Membran bildende Material hineinerstrecken und/oder benachbarte Mikroporen miteinander verbinden.

Gemäß der vorliegenden Erfindung ist mindestens eine funktionelle Gruppe an die Membran gebunden. Dabei sind die funktionellen Gruppen Liganden, die befähigt sind, mit in Medien enthaltenen Adsorbenden Wechselwirkungen einzugehen. Insbesondere kann der Ligand ein Katalysator sein, oder es können mindestens zwei strukturell unterschiedliche Liganden an die Membran gebunden sein.

Erfindungsgemäß kann das Trennen des mindestens einen flächigen Adsorbens von dem flüssigen Medium durch Druckfiltration, Vakuumfiltration, Zentriguation oder unter Schwerkrafteinwirkung erfolgen.

Gemäß der vorliegenden Erfindung kann die Komponente mit dem höheren mittleren Molekulargewicht ein mittleres Molekulargewicht von mindestens 50.000 haben, bevorzugt von mindestens 100.000.

Des Weiteren kann erfindungsgemäß das mindestens eine flächige Adsorbens mit mindestens einer mikroporösen, oberflächenmodifizierten und adsorptiven Membran kombiniert werden.

Im Stand der Technik sind Verfahren zur Entfernung von Kontaminanten in der sogenannten negativen Adsorption bekannt. In solchen Verfahren werden Bedingungen in den Medien angestrebt (z. B. pH, Leitfähigkeit), die die Bindung der Kontaminanten an das Adsorbens ermöglichen, ohne das Zielmolekül an das Adsorbens zu binden. Bei der Reinigung von monoklonalen Antikörpern werden oft Anionenaustauscher in Form von Membranen oder Gelen für die Kontaminantenentfernung (z.B. DNA, Viren, Wirtzellproteine, leached Protein A, Aggregate oder Endotoxine) eingesetzt. Bei diesen Anwendungen sollte der pH-Wert des antikörperhaltigen Mediums unter dem isoelektrischen Punkt des Antikörpers liegen, damit der Antikörper die gleiche (positive) Ladung wie der Anionenaustauscher trägt und somit nicht bindet. Ein solches Verfahren ermöglicht aber nicht die Entfernung von Kontaminanten, die unter den gewählten Bedingungen keine oder die gleiche Ladung tragen wie der Antikörper.

Ähnlich können auch Kationenaustauscher für die Kontaminantenentfernung eingesetzt werden. Auch hier muss bei den im Stand der Technik bekannten Kationenaustauschern darauf geachtet werden, dass der pH-Wert oberhalb des isoelektrischen Punktes des Antikörpers liegt. Ein solches Verfahren hat mehrere Nachteile: Zum Einen können Antikörper bei höheren pH-Werten instabil sein, was zu Verlusten des Produktes führen kann, zum Anderen werden Kontaminanten, die unter den gewählten Bedingungen keine oder eine gleiche Ladung tragen wie der Antikörper, nicht abgereichert.

Neue Verfahren mit verbesserten Trennungseigenschaften werden für die Reinigung von biopharmazeutischen Produkten gebraucht.

Das erfindungsgemäße Verfahren zur Stofftrennung wird durch die Porenmorphologie einer Membran ermöglicht, die eine poröse Doppelstruktur aufweist, welche besteht aus:

- Mikroporen mit einem Durchmesser im Bereich von >100 nm bis 20 $\mu$m und
- Ultraporen mit einem Durchmesser von < 100 nm, die für Dextranblau mit einem mittleren Molekulargewicht Mw von 2.000.000 nicht zugänglich sind und wobei der Anteil des Volumens der Ultraporen an dem für Wasser zugänglichen Gesamtporenvolumen mehr als 15% beträgt.

Das erfindungsgemäße Verfahren unter Einsatz einer derartigen, bevorzugt Kationen austauschenden Membran ermöglicht es, Bedingungen zu wählen, unter denen der Antikörper an die Membran binden kann, aber aufgrund seiner Größe nur wenige Bindungstellen in den Mikroporen der Membran besetzt. Die Antikörpermengen, die gebunden werden können, liegen unter 0,1% der Gesamtmenge und können so vernachlässigt werden. Da die Bindungsstellen in den Ultraporen der Membran frei bleiben, können kleinere, gleich geladene Kontaminanten gebunden und abgereichert werden.

Das erfindungsgemäße Verfahren kann auch mit den bekannten Verfahren kombiniert werden, was zur deutlichen Verbesserung der Kontaminantenentfernung führt. Zum Beispiel können die Kationenaustauschermembranen mit einer Anionenaustauschermembran in einer oder mehreren Vorrichtungen kombiniert werden, so dass die Abreicherung eines breiten Spektrums an Kontaminanten gewährleistet wird.

In einem anderen Beispiel können bei der Virenaufreinigung z. B. kleinere, geladene Kontaminanten, wie DNA-Bruchstücke, in den Ultraporen einer Anionenaustauschermembran mit der genannten Porenmorphologie aus Mikro- und Ultraporen gebunden werden, wobei die großen, gleich geladenen Virenmoleküle aufgrund der Größenausschlußgrenze der Ultraporen durch die Mikroporen hindurchfließen und praktisch nicht in der inneren Oberfläche der Ultraporen gebunden werden.

Die angegebenen Beispiele sollten den Einsatz des erfindungsgemäßen Verfahrens erläutern, bedeuten aber keine Beschränkung der Applikationsmöglichkeiten.

Als Ausgangsmaterial für die in dem erfindungsgemäßen Verfahren eingesetzte Adsorptionsmembran dient eine Celluloseester-Membran, die mit mindestens einer Lösung unter Bedingungen in Kontakt gebracht wird, die zum einen zu einer Quellung der Celluloseester-Matrix führen und zum anderen gleichzeitig, d.h. in situ, zur Hydrolyse (Verseifung) der Estergruppen zu Hydroxylgruppen, wobei eine Cellulosehydrat-Membran entsteht.

Die Quellung der Celluloseester-Matrix während der Hydrolyse (Verseifung) der Estergruppen wird durch den Quellungsgrad, d.h. das Verhältnis der Wasserpermeabilität der zuvor mit Wasser benetzten Celluloseester-Membran zur Wasserpermeabilität der endgültigen, d.h. verseiften, aktivierend vernetzten und gegebenenfalls mit Ligand(en) versehenen Cellulosehydrat-Membran, beschrieben.

Anschließend an die Verseifung wird die erhaltene Cellulosehydrat-Matrix durch Umsetzung der Hydroxylgruppen mit einem oder mehreren, mindestens bifunktionellen Reagens vernetzt und danach werden in die vernetzte Matrix funktionelle Gruppen (Liganden) zur Befähigung der adsorptiven Stofftrennung eingeführt.

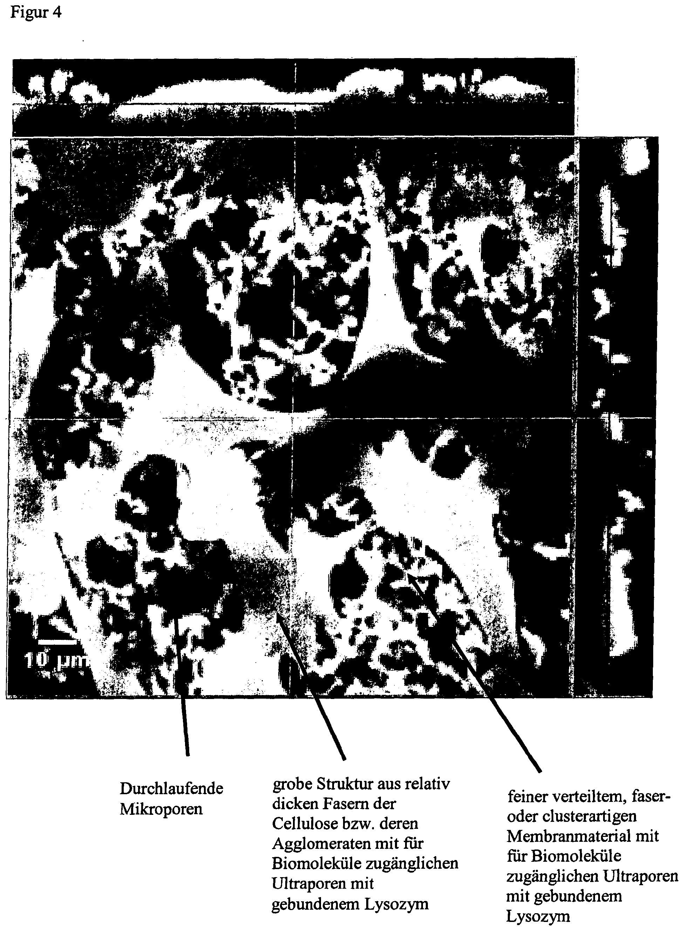

Überraschenderweise wurde festgestellt, daß die Bindungskapazität der Cellulosehydrat-Membran deutlich erhöht wird, wenn der Verseifungsschritt unter Bedingungen durchgeführt wird, unter denen die Cellulose quellen kann. Die Erhöhung der Bindungskapazität für Biomoleküle könnte auf die erhöhte Anzahl an für Biomoleküle zugänglichen, amorphen Bereichen der Cellulose zurückgeführt werden. Durch die Quellung des Celluloseträgers entstehen zwei Arten von Poren: a) Mikroporen mit einem Durchmesser von >100 nm, die in der Regel kleiner als die Originalporen der Celluloseester-Membran sind, und b) Ultraporen (amorphe Bereiche der Cellulose) mit einem Durchmesser von < 100 nm, die dergestalt sind, daß sie für Dextranblau (erhältlich als Blue Dextran molecular weight 2,000,000 von der Firma Sigma, St. Louis, Missouri, USA, Produktnummer D5751, CAS-Nummer: 87915-38-6) nicht zugänglich sind und die eine zusätzliche, für Liganden und Adsorbenden zugängliche Adsorptionsfläche anbieten (vgl. Figur 1c). Die Adsorptionswirksamkeit der erfindungsgemäßen Membran ist nicht auf die Phasengrenzfläche der verbundenen Mikroporen mit dem Medium beschränkt, sondern erstreckt sich zumindest auf einen Teil oder sogar das gesamte Volumen in den Ultraporen des Trägers (siehe Figur 4). Figur 4 zeigt eine konfokale Mikroskopieaufnahme einer erfindungsgemäßen Membran mit Ultraporen.

Die Quellung der Cellulose während der Verseifung kann durch eine geeignete Vorbehandlung des Celluloseesters oder durch die Parameter der Verseifung (Zusammensetzung des Verseifungsmediums, Art des Additivs, Konzentration des Additivs, Verseifungstemperatur) beeinflußt und gesteuert werden. So kann die Permeabilität und Kapazität der Membran eingestellt werden. Die in dem erfindungsgemäßen Verfahren hergestellten, adsorptiven Cellulosehydrat-Membranen zeigen gegenüber den durch auf dem Fachgebiet bekannten Herstellungsverfahren hergestellten Cellulosehydrat-Membranen deutlich höhere Bindungskapazitäten bei vergleichbaren Permeabilitäten.

Wie im Weiteren beschrieben wird, kann das Verfahren zur Herstellung der erfindungsgemäß verwendeten Membran in drei Schritten durchgeführt werden, wobei die Einstellung des gewünschten Quellungsgrads, des Durchflusses und der Bindungskapazität sowohl durch die Parameter der Vorbehandlung (Art des Additivs, Konzentration des Additivs, Vorbehandlungstemperatur) als auch die Parameter der Verseifung (Zusammensetzung des Verseifungsmediums, Art des Additivs, Konzentration des Additivs, Verseifungstemperatur) gesteuert werden kann. Die erfindungsgemäße Membran kann auch ohne Vorbehandlung der Celluloseester-Matrix hergestellt werden. Hohe Quellungsgrade der Cellulosehydrat-Matrix können gemäß dem erfindungsgemäßen Verfahren durch hohe Konzentration an Alkalimetallhydroxid im Verseifungsmedium, hohe Konzentration an wasserstoffbrückenbrechenden Verbindungen oder niedrige Temperatur des Verseifungsmediums erreicht werden.

Durch die Art des Vernetzungsmittels, die Konzentration des Vernetzungsmittels, die Konzentration des Vernetzungskatalysators, die Vernetzungsdauer, gegebenenfalls die Art und die Konzentration eines inerten organischen Lösungsmittels und/oder die Vernetzungstemperatur kann der Vernetzungsgrad, die Porengröße und die Anzahl an restlichen, aktiven Gruppen, z.B. Epoxid-Gruppen, gesteuert werden. Dadurch kann die für die Bindung der funktionellen Gruppen oft notwendige Aktivierung bereits in dem Vernetzungsschritt stattfinden.

In einem weiteren Schritt werden funktionelle Gruppen, z.B. an die Hydroxylgruppen, der vernetzten Membran gebunden. Methoden zur Bindung von funktionellen Gruppen sind dem Fachmann an sich bekannt (z.B. aus Greg T.

Hermanson, A. Krishna Mallia, Paul K. Smith, Immobilized Affinity Ligand Techniques, Academic Press, INC, 1992).

Bevorzugt werden funktionelle Gruppen über Epoxid-Gruppen oder Aldehyd-Gruppen an die Cellulosemembran gebunden. Die Einführung der Epoxid-Gruppen kann bereits in dem Vernetzungsschritt oder nachträglich stattfinden.

Die Kombinationen der Einflußgrößen (a) der Herstellungsbedingungen der als Ausgangsmaterial verwendeten Celluloseester-Membran, (b) der Bedingungen der gegebenenfalls durchgeführten Vorbehandlung, (c) der Verseifungsbedingungen und (d) der Vernetzungsbedingungen der Celluloseester-Membran, ermöglichen es auch, aus einer Ausgangsmembran mehrere unterschiedliche Endprodukte herzustellen, was produktionstechnisch eine erhebliche Vereinfachung zur Folge hat.

## Ausgangsmembran

Die in dem erfindungsgemäßen Verfahren als Ausgangsmembran verwendete Celluloseester-Membran mit einer Porengröße von 0,1 bis 20 $\mu$m, vorzugsweise 0,5 bis 15 $\mu$m und mehr bevorzugt von 1 bis 10 $\mu$m wird durch ein übliches, auf dem Fachgebiet bekanntes Herstellungsverfahren hergestellt. Zur Bestimmung der Porengröße wird ein "Capillary Flow Porometry Test" durchgeführt. Weitere Details sind der Bedienungsanleitung (Capillary Flow Porometer 6.0, CAPWIN Software System, Fa. Porous Materials Inc.) zu entnehmen. Celluloseester-Membranen können aus Cellulosemonoacetat, Cellulosediacetat, Cellulosetriacetat, Cellulosepropionat, Cellulosebutyrat und Celluloseacetobutyrat oder anderen geeigneten Celluloseestern oder Cellulosenitrat, Methylcellulose oder Ethylcellulose, sowie Gemischen davon, aufgebaut sein, wobei Celluloseacetate, insbesondere Cellulosediacetat, bevorzugt sind bzw. ist. Dem Fachmann ist bekannt, daß die Celluloseester-Membran zum Teil auch Hydroxylgruppen neben den Estergruppen enthalten kann.

## Vorbehandlung

Vor dem Verseifen kann die Celluloseester-Membran in einem geeigneten Medium vorbehandelt werden. Die Temperatur in dem Vorbehandlungsschritt liegt vorzugsweise in einem Bereich von 20 bis 100 °C, wobei eine Temperatur in einem Bereich von etwa 60 °C bis etwa 80 °C besonders bevorzugt ist. Als Vorbehandlungsmedium kann ein Gas, wie beispielsweise Luft, ein organisches Lösungsmittel, wie beispielsweise ein Alkohol, oder ein wässriges Medium verwendet werden, wobei ein wässriges Medium bevorzugt ist. Das Vorbehandlungsmedium enthält vorzugsweise ein oder mehrere Additiv(e), das bzw. die eine gegenüber einem Celluloseester lösende oder weichmachende Wirkung aufweist bzw. aufweisen. Geeignete Additive sind vor allem Säuren, insbesondere Carbonsäuren, wie Essigsäure, und wasserlösliche Weichmacher für Celluloseester, wie Diacetin, Triacetin und Sulfolan. Es ist jedoch vor allem aus wirtschaftlichen Gründen besonders bevorzugt, Essigsäure als Additiv für das Vorbehandlungsmedium zu verwenden, obwohl auch Diacetin und Triacetin ausgezeichnete Ergebnisse liefern, jedoch teurer sind. Die Konzentration des Additivs in dem Vorbehandlungsmedium unterliegt keinen besonderen Beschränkungen.

Die Dauer der Vorbehandlung hat keinen wesentlichen Einfluß auf den Vorbehandlungseffekt, sofern eine Mindesteinwirkdauer angewendet wird, die eine Temperaturangleichung der Celluloseester-Membran in dem Vorbehandlungsmedium und eine Konzentrationsangleichung des gegebenenfalls verwendeten Additivs in der Membran gewährleistet. Die obere Grenze der Einwirkungsdauer des Vorbehandlungsmediums ist durch jene Zeit bestimmt, ab der eine chemische Umsetzung der Celluloseestermembran mit dem Vorbehandlungsmedium, beispielsweise durch Hydrolyse, eintreten könnte. In anderen Worten ausgedrückt, wird die Einwirkungsdauer des Vorbehandlungsmediums derart eingestellt, daß keine (frühzeitige) Hydrolyse oder Verseifung der vorbehandelten Celluloseester-Membran eintritt. Üblicherweise beträgt die Einwirkungsdauer des Vorbehandlungsmediums auf die Celluloseester-Ausgangsmembran zwischen 0,1 Sekunden und 1 Stunde, wobei eine Einwirkungsdauer von 10 Sekunden bis 10 Minuten bevorzugt ist. Das Ausmaß des Vorbehandlungseffekts ist abhängig von der höchsten Temperatur in Verbindung mit der höchsten Konzentration des Additivs, die auf die Celluloseester-Membran einwirken. Wenn also die Abkühlung oder Ausspülung des Additivs über einen längeren Zeitraum erfolgt, ist das ohne Einfluß auf den bereits erreichten Vorbehandlungseffekt. Die Beendigung der Vorbehandlung kann daher durch Ausspülen des Vorbehandlungsadditivs aus der Membran und/oder Absenkung der Temperatur des Vorbehandlungsmediums erfolgen.

## Verseifung

Die gegebenenfalls vorbehandelte Celluloseester-Membran wird mit einem geeigneten Verseifungsmedium verseift, wodurch sich die Cellulosehydrat-Membran unter Quellung der Cellulose-Matrix ausbildet. Je nach der Art des Vorbehandlungsmediums kann die Celluloseester-Membran trocken oder naß im Verseifungsschritt eingesetzt werden.

Durch die Quellung der Cellulose wird die Zugänglichkeit der Hydroxylgruppen für die Anbindung der funktionellen Gruppen und anschließend für die Adsorbenden verbessert. Das Verseifen der Cellulose-Ester-Membran wird in einem wässrigen Medium mit einem pH-Wert von >7, d.h. ein basisches Medium, durchgeführt, wobei das Verseifungsmedium Natrium- oder Lithiumhydroxid enthält. Es können auch Gemische aus Natrium- oder Lithiumhydroxid und anderen alkalischen Verbindungen wie Alkalimetallcarbonat, wie Natriumcarbonat, Kaliumcarbonat, Cäsiumcarbonat, Natriumhydrogencarbonat, und/oder Natriumtriphosphat, Kaliumtriphosphat, Natriumsilikat und Kaliumsilicat eingesetzt werden.

Die Konzentration des Natrium- oder Lithiumhydroxids im Verseifungsmedium beträgt 0,4 bis 50 Gew.-%, bezogen auf das Verseifungsmedium, und besonders bevorzugt 0,4 bis 10 Gew.-%. Gemäß einer besonders bevorzugten Ausführungsform der vorliegenden Erfindung wird ein Verseifungsmedium aus Wasser und Natriumhydroxid verwendet, wobei die Konzentration des Natriumhydroxids in dem Verseifungsmedium in einem Bereich von 0,4 bis 20 Gew.-%, besonders bevorzugt in einem Bereich von 0,4 bis 4 Gew.-% liegt.

Das Verseifungsmedium kann ein oder mehrere Additiv(e) enthalten, das bzw. die eine gegenüber einem Celluloseester quellungsbeeinflussende Wirkung aufweist bzw. aufweisen. Geeignete Additive sind vor allem Salze, wie Natriumchlorid, Natriumsulfat und Natriumacetat, Wasserstoffbrücken brechende Verbindungen, wie Harnstoff oder organische Lösungsmittel, wie Ethylamin. Das organische Lösungsmittel wird vorzugsweise aus der Gruppe von Alkohlen, Ketonen oder Ethern ausgewählt. Besonders bevorzugt ist Ethanol, Methanol, Ethylenglycol, Propylenglycol, Glycerin, Aceton, Dioxan oder Diglyme. Das Additiv in dem Verseifungsmedium sollte die Quellung beeinflussen, aber nicht vollständig unterdrücken.

Die Temperatur des verwendeten Mediums in dem Verseifungsschritt kann von etwa 10 °C bis zum Siedepunkt des Verseifungsmediums betragen, wobei eine Temperatur in einem Bereich von 15 °C bis etwa 25 °C bevorzugt ist.

Die Verseifungsdauer richtet sich nach der Zusammensetzung des Verseifungsmediums und der Verseifungstemperatur. Üblicherweise beträgt die Verseifungsdauer 0,1 bis 60 Minuten, wobei eine Verseifungsdauer von 5 bis 45 Minuten bevorzugt ist. Besonders bevorzugt ist eine Verseifungsdauer von 20 bis 40 Minuten.

## Vernetzung

Die nach der gegebenenfalls durchgeführten Vorbehandlung und der unter Quellung stattgefundenen Verseifung erhaltene Cellulosehydrat-Membran wird mit einem Vernetzungsmittel zur Erhöhung der chemischen Widerstandsfähigkeit der Membran und/oder zur Einführung von funktionellen Gruppen vernetzt.

Das Vernetzungsmittel weist mindestens zwei funktionelle Gruppen im Molekül auf, die mit den Hydroxylgruppen von Cellulose reaktiv sind und somit eine Vernetzung von Cellulose ermöglichen. Die verwendbaren Vernetzungsmittel unterliegen grundsätzlich keinen besonderen Beschränkungen und ein Fachmann ist in der Lage, sie aus einer Reihe von für die Vernetzung von Cellulose verwendbaren Vernetzungsmitteln auszuwählen. Es ist jedoch bevorzugt, in dem Vernetzungsschritt eine Diepoxidverbindung oder auch andere, mit Hydroxylgruppen von Cellulose reaktive Verbindungen mit mindestens zwei reaktiven, funktionellen Gruppen, wie Diisocyanat, Epichlorhydrin, Epibromhydrin, Dimethylharnstoff, Dimethylethylenharnstoff, Dimethylchlorsilan, Bis(2-hydroxyethylsulfon), Divinylsulfon, Alkylen-Dihalogen, Hydroxyalkylen-Dihalogen und Glycidylether zu verwenden.

Aus der Gruppe der Glycidylether sind 1,4-Butandioldiglycidylether, Ethylenglycoldiglycidylether, Glycerindiglycidylether und Polyethylenglycoldiglycidylether bevorzugt.

Besonders bevorzugt ist die Verwendung von 1,4-Butandioldiglycidylether oder Epichlorhydrin als Vernetzungsmittel.

Optional kann ein Gemisch von unterschiedlichen Vernetzungsmitteln verwendet werden.

Die Vernetzung kann in einem wässrigen Medium, in einem organischen Lösungsmittel oder auch in einem Gemisch aus Wasser und einem organischen Lösungsmittel stattfinden. Vorzugsweise wird die Vernetzung in einem wässrigen Medium durchgeführt.

Ferner ist es bevorzugt, einen Vernetzungskatalysator, wie Natriumhydroxid, zur Beschleunigung der Vernetzung von Cellulose mit dem Vernetzungsmittel zu verwenden.

Die Temperatur des verwendeten Mediums in dem Vernetzungsschritt kann von etwa 4 °C bis zum Siedepunkt des Vernetzungsmediums betragen, wobei eine Temperatur in einem Bereich von 5 °C bis etwa 70 °C bevorzugt ist. Besonders bevorzugt ist eine Temperatur von 20 °C bis 40 °C.

Üblicherweise beträgt die Vernetzungsdauer 10 Minuten bis 100 Stunden, wobei eine Verseifungsdauer von 30 Minuten bis 48 Stunden bevorzugt ist. Besonders bevorzugt ist eine Verseifungsdauer von 2 bis 24 Stunden.

Wie vorstehend beschrieben, kann das Verfahren zur Herstellung der erfindungsgemäßen Membran in drei Schritten durchgeführt werden, wobei die Einstellung des gewünschten Quellungsgrads der Matrix sowohl durch die Parameter der Vorbehandlung (Art des Additivs, Konzentration des Additivs, Vorbehandlungstemperatur) als auch die Parameter der Verseifung (Zusammensetzung des Verseifungsmediums, Art des Additivs, Konzentration des Additivs, Verseifungstemperatur) gesteuert werden kann. Die erfindungsgemäße Membran kann auch ohne Vorbehandlung hergestellt werden.

**Aktivierung und Bindung von Liganden**

In einem weiteren Schritt werden Sulfonsäureliganden an die Hydroxylgruppen der vernetzten Cellulosehydrat-Membran gebunden. Methoden zur Bindung von funktionellen Gruppen, wie beispielsweise Sulfonsäureliganden, sind dem Fachmann bekannt (z.B. aus Greg T. Hermanson, A. Krishna Mallia, Paul K. Smith, Immobilized Affinity Ligand Techniques, Academic Press, INC, 1992).

Bevorzugt werden die ionischen und/oder hydrophoben Liganden über Epoxid-Gruppen oder Aldehyd-Gruppen an die Cellulosemembran gebunden. Die Epoxid-Aktivierung kann bereits in dem Vernetzungsschritt oder nachträglich stattfinden.

Es ist auch möglich, die Liganden während der Vernetzung einzuführen, z.B. durch Zugabe von einem Amin und/oder von einer monofunktionellen Epoxidverbindung, wie Phenylglycidylether oder Butylglycidylether zur Diepoxidverbindung.

Die erfindungsgemäßen Membranen können nach der Einführung der Liganden optional getrocknet werden. Membranen können direkt aus Wasser oder organischen Lösungsmitteln, bevorzugt Alkohol, getrocknet werden, oder nach einem stufenweise durchgeführten Austausch des Wassers durch ein organisches Lösungsmittel. Bevorzugt werden die Membranen aus einem Medium getrocknet, das eine porenstabilisierende Verbindung enthält. Besonders bevorzugt werden die erfindungsgemäßen Membranen aus wässriger Glycerinlösung getrocknet. Die Konzentration des Glycerins liegt vorzugsweise im Bereich von 5 bis 40 Gewichtsprozent, bezogen auf die wässrige Lösung.

**Erläuterung der Beispiele**

Vernetzte Cellulosehydrat-Membranen mit niedrigem Quellungsgrad werden beispielsweise aus mit ethanolischer Kalilauge verseiften Celluloseester-Membranen hergestellt. Eine Celluloseacetat-Membran ergibt auf diese Weise eine vernetzte Cellulosehydrat-Membran mit geringfügig niedrigem Durchfluss (siehe Beispiel 1), die aber nach Einführung von Liganden praktisch kein Adsorptionsvermögen aufweist (siehe Tabelle 3).

Es wurde nun gefunden, daß bei Verseifung mit wässriger Natronlauge zwar der Durchfluß sinkt (siehe Beispiel 2), nach Belegung mit diversen Liganden aber deutlich erhöhte Bindungskapazitäten auftreten, wobei steigende NatronlaugeKonzentrationen sich in Richtung einer stärkeren Durchflußminderung und höheren Bindungskapazitäten auswirken (siehe Tabelle 3). Gegenüber den durchgehenden Mikroporen, die sich bei der Verseifung mit ethanolischer Kalilauge vorwiegend bilden, scheint bei der Verseifung mit wässriger Natronlauge die Entstehung einer Vielzahl kleiner Ultraporen bevorzugt zu sein. Eine höhere Verseifungstemperatur sowie ein zusätzlicher Gehalt an Elektrolyten einschließlich bei der Verseifung bereits gebildeten Natriumacetats wirken sich dabei in der gleichen Richtung aus, wie eine niedrigere Natronlauge-Konzentration.

In WO 2007/017085 A2 wird ein Verfahren zur Herstellung von vernetzten Cellulosehydrat-Membranen beschrieben, das in der simultanen Verseifung und Vernetzung von Celluloseester-Membranen besteht und für Filtrations- und Adsorptionsmembranen gleichermaßen geeignet sein soll. Eines der Ziele der dort beschriebenen Erfindung ist die Verseifung und Vernetzung des Celluloseesters unter Bedingungen, die die Struktur und Permeabilität der Membran nicht beeinflussen. Durch gleichzeitige Verseifung und Vernetzung unter Bedindungen, die die Quellung und Strukturänderung unterdrücken ($Na_2SO_4$, niedrige Natronlauge-Konzentration) wird keine signifikante Bindungskapazität festgestellt (siehe Vergleichsbeispiel 2). Erst wenn die Laugenkonzentration erhöht wird, kommt es zur Erhöhung der Bindungskapazität. Durch die Quellung der Cellulose kommt es hier aber auch zur Änderung der Porenstruktur, was im Widerspruch zum in dem Stand der Technik beschriebenen, simultanen Verseifungs- und Vernetzungsprozess steht. Die Bindungskapazität beträgt hier aber nur ca. 5 % der Bindungskapazität im Vergleich zu der separat durchgeführten Verseifung und Vernetzung (siehe Beispiel 2 und Vergleichsbeispiel 2).

Weiterhin wurde gefunden, daß sich die unterschiedliche Vorbehandlung der Celluloseacetat-Membran unterschiedlich auf die Eigenschaften der erfindungsgemäßen adsorptiven Membran auswirkt. Der Durchfluss sinkt und die Bindungskapazität steigt, wenn die Celluloseacetat-Membran vor der Verseifung auf 80 °C an der Luft erhitzt wurde (siehe Beispiel 3). Wenn die Celluloseacetatmembran vor der Verseifung in 20%iger Essigsäure auf 80 °C erhitzt wird (siehe Beispiel 4) steigt der Durchfluss im Vergleich zu der nicht vorbehandelten Membran aus dem Beispiel 2 und die Bindungskapazität ändert sich abhängig von der Größe des Proteins. Für Lysozym (MG: 14,3 kDa) steigt die Bindungskapazität, für Rinderserumalbumin (RSA; MG: 60 kDa) und $\gamma$-Globulin (MG: 150 kDa) sinkt die Bindungskapazität. Die Ultraporen in der Cellulosematrix werden kleiner, und die Selektivität der Membran wird höher. Die Selektivität ist hier definiert als das Verhältnis der Bindungskapazität (Kap) für ein Protein, z.B. Lysozym (Lys), zur Bindungskapazität (Kap) für ein anderes Protein, z.B. $\gamma$-Globulin (Glob), ausgedrückt in $mg/cm^2$ Membranfläche, also z.B. der Quotient KapLys/KapGlob.

Je höher die Selektivität ist, umso stärker wirkt sich neben der Adsorption auch der Größenausschlußeffekt aus. Die Selektivität kann auch durch die Bindungsbedingungen, wie z.B. den pH-Wert oder die Salzkonzentration, beeinflußt werden. So ist die Selektivität in 10 mM Kaliumphosphat (KPi)-Puffer mit einem pH-Wert von 7,0 höher als die Selektivität in 20 mM Natriumacetat + 50 mM NaCl-Puffer mit einem pH-Wert von 5,0. Gegenüber einer im Stand der Technik bekannten Membran, z.B. einer Sartobind® S Membran der Fa. Sartorius Stedim Biotech GmbH, ist die Selektivität einer erfindungsgemäßen Membran (vgl. Bsp. 4) um bis zu 8 mal höher.

In der folgenden Übersicht sind die Bindungskapazitäten und Selektivitäten der erfindungsgemäßen Membranen der Beispiele 2 und 4 im Vergleich mit einer Standardmembran (Sartobind® S Membran) bezüglich der Proteine Lysozym und γ-Globulin unter verschiedenen Betriebsbedingungen wiedergegeben.

| Membran | Protein | Bindungspuffer | Bindungskapazität [mg/cm²] | Selektivität[3] |
|---|---|---|---|---|
| Beispiel 2 | Lysozym | A[1] | 2,82 | 14,8 |
| | γ-Globulin | A | 0,19 | |
| | Lysozym | B[2] | 1,93 | 4,4 |
| | γ-Globulin | B | 0,44 | |
| Beispiel 4 | Lysozym | A | 3,17 | 24,4 |
| | γ-Globulin | A | 0,13 | |
| | Lysozym | B | 2,23 | 9,7 |
| | γ-Globulin | B | 0,23 | |
| Sartobind® S | Lysozym | A | 1,13 | 3,1 |
| | γ-Globulin | A | 0,36 | |
| | Lysozym | B | 1,00 | 1,3 |
| | γ-Globulin | B | 0,75 | |
| [1] Bindungspuffer A:10 mM KPi-Puffer mit pH 7,0 | | | | |
| [2] Bindungspuffer B:20 mM Natriumacetat + 50 mM NaCl mit pH 5,0 | | | | |
| [3] Selektivität ist definiert als das Verhältnis der Bindungskapazität für Lysozym zur Bindungskapazität für γ-Globulin ausgedrückt in mg/cm² Membranfläche | | | | |

Die Vorbehandlung kann beispielsweise bei speziellen Trennaufgaben dann vorteilhaft sein, wenn die Spezifität des Liganden für die Stofftrennung alleine nicht ausreicht und die Molmassen der zu trennenden Komponenten so stark unterschiedlich sind, daß sich aus der Überlagerung der rein adsorptiven Stofftrennung mit einem Größenausschlußeffekt insgesamt eine Verbesserung der Trennleistung ergibt, und die Beeinflussung der Porengröße der Ultraporen durch die Wahl der Base und deren Konzentration einer Unterstützung bedarf. Eine vollständige Trennung auf der Basis allein dieses Effekts ist allerdings nicht möglich, weil der Größenausschluß nur für die Adsorption an der inneren Oberfläche der Ultraporen wirksam wird, nicht aber an der äußeren Oberfläche der Mikroporen.

Diese Befunde lassen bei der Verseifung und Vernetzung von Celluloseacetaten auf komplexe Quellungs- und Entquellungsvorgänge schließen, deren Auswirkungen im Hinblick auf die Struktur des Endprodukts schwer zusammenfassend darzustellen sind, weil es sowohl Vorgänge gibt, bei denen eine Durchflussminderung mit einer Erhöhung der Bindungskapazität gekoppelt ist, als auch solche, bei denen das nicht der Fall ist. Die erstgenannten werden im folgenden als "produktiv" bezeichnet, die sonstigen als "unproduktiv". Die Vorbehandlung der Celluloseacetat-Membran wirkt sich unterschiedlich auf die Änderung der Porenstruktur, die Bildung von Mikroporen sowie von Ultraporen aus. So ist es möglich, durch geeignete Wahl der Vorbehandlung den Durchfluss, die Bindungskapazität aber auch den Größenausschluss der erfindungsgemäßen adsorptiven Membran zu beeinflussen. Das Hauptziel des erfindungsgemäßen Verfahrens ist die Beschränkung auf produktive Durchflussminderungen, wobei nicht nur das Quellverhalten des Ausgangsmaterials, der Celluloseacetat-Membran, und des Endprodukts, der vernetzten Cellulosehydrat-Membran, in Betracht zu ziehen sind, sondern auch das gesamte Spektrum der Zwischenprodukte im teilverseiften und teilvernetzten Zustand. So ist beispielsweise bekannt, daß Celluloseacetate abnehmenden Acetylgehalts in einem engen Bereich sogar einen Zustand der Wasserlöslichkeit durchlaufen.

Erfindungsgemäß wird eine Celluloseester-Membran sequentiell in einem quellenden Medium verseift, mit einem mindestens bifunktionellen Agens vernetzt und mit einem adsorptionswirksamen Liganden versehen. Das Quellvermögen der Alkalimetallhydroxide nimmt in Richtung kleinerer Kationenradien und höherer Konzentrationen zu (siehe Beispiel 5).

Die Vernetzung der Cellulose erfolgt erfindungsgemäß vorzugsweise mit 1,4-Butandioldiglycidylether. In einer Ausgestaltung der Erfindung erfolgt die Vernetzung der Cellulose mit 1,4-Butandioldiglycidylether in der Weise, dass infolge einer zum Teil einseitigen Reaktion eine ausreichende Zahl nicht umgesetzter Epoxidgruppen erhalten bleibt ("aktivierende Vernetzung", siehe Beispiel 2), die zur Bindung oder zur Ankopplung bzw. zum Aufbau von Liganden dienen können. Die nicht umgesetzten Epoxidgruppen sind relativ hydrolysestabil und wurden noch nach bis zu 24 Stunden Feuchtlagerung bei Raumtemperatur für Folgereaktionen eingesetzt. In einer anderen Ausführungsform der Erfindung zur Bindung "aktiver" Liganden wird die Vernetzung unter verschärften Bedingungen (längere Vernetzungsdauer und/oder höhere Alkalikonzentration und/oder höhere Temperatur) durchgeführt, so dass unter gesteigerter Reaktion mit der Cellulose und/oder gesteigerter Hydrolyse der überzähligen Gruppen ein Verbleib von Epoxidgruppen weitgehend unterbleibt ("nicht aktivierende Vernetzung", siehe Beispiel 6). Restliche Epoxidgruppen können auch durch nachträgliche Behandlung mit z.B. 5%iger Schwefelsäure bei erhöhter Temperatur hydrolisiert werden.

Der Durchfluß der erfindungsgemäß verwendeten Membran nach Beispiel 2 für 20 mM tris/HCl Puffer mit einem pH-Wert von 7,3 ist um 8 % größer als der für reines Wasser. Entsprechende Werte von Adsorptionsmembranen, die nach dem Stand der Technik durch Beschichtung hergestellt wurden, liegen zwischen 20 % im Fall eines vernetzten Hilfspolymeren und 200 % bei einem unvernetzten. Die entstandene Porenstruktur gibt sich durch die geringe Abhängigkeit des Durchflusses von der Ionenstärke des Mediums als ein Hybrid aus Aerogel und Xerogel ähnlich wie bei einem vernetzten Agarosegel zu erkennen. Das steht im Einklang mit der Tatsache, daß auch die Einführung von hydrophoben Liganden zu einer leistungsfähigen Adsorptionsmembran für die hydrophobe Interaktionschromatographie (HIC) führt (siehe Beispiele 4 und 10).

Die erfindungsgemäß verwendeten Adsorptionsmembranen lassen sich von nach dem Stand der Technik durch Polymerbeschichtung oder Pfropfung hergestellten Adsorptionsmembranen durch Rasterelektronenmikroskopie schwer unterscheiden, weil deren Auflösungsvermögen durch die den Hauptunterschied ausmachenden kleinporigen Strukturen (d.h. Ultraporen) überfordert wäre. Dagegen liefert die Charakterisierung von adsorptiven Membranen mittels der sogenannten konfokalen Laserfluoreszenzmikroskopie (CLSM) unter geeigneten Bedingungen simultan sowohl Information zur Porenstruktur als auch zur Verteilung von an funktionellen Gruppen gebundenem Protein in der Membran. Dafür müssen Membranmaterial und Protein mit zwei verschiedenen Fluoreszenzfarbstoffen markiert werden. Alle mikroskopischen Messungen wurden ungefähr im selben Abstand (ca. 20 $\mu$m) von der jeweiligen äußeren Oberfläche durchgeführt. In allen Fällen führten drei unabhängige Messungen an verschiedenen x, $\gamma$-Positionen zu sehr ähnlichen, für den jeweiligen Membrantyp charakteristischen Ergebnissen.

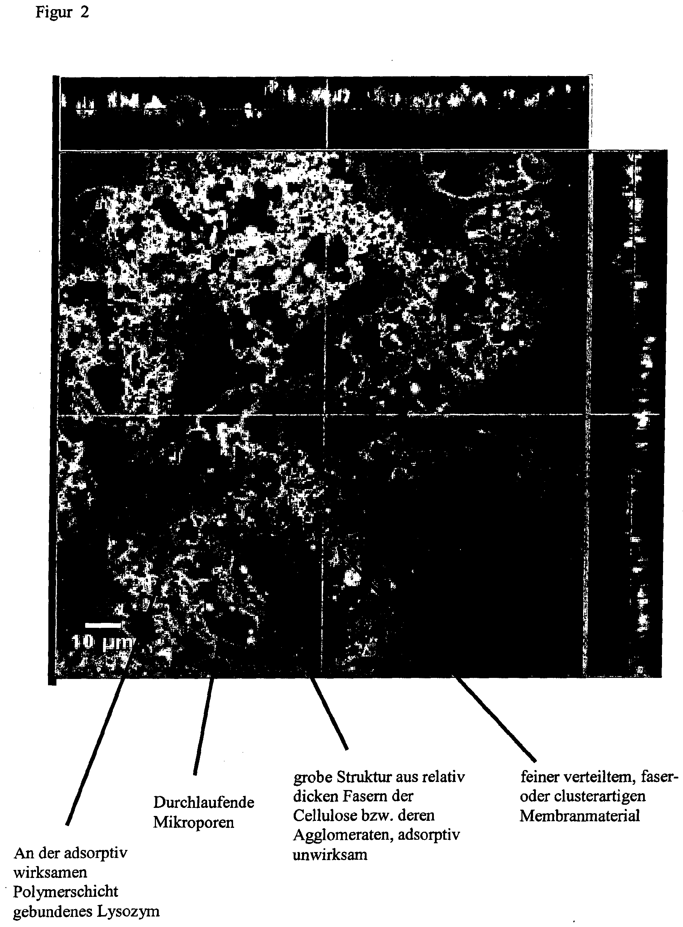

Charakteristisch für alle Membranproben ist eine sehr grobe Struktur (dunkle Bereiche in den Figuren 2 - 4) aus relativ dicken Fasern bzw. deren Agglomeraten mit feiner verteiltem Faser- oder clusterartigen Membranmaterial mit dazwischen sich befindlichen, vollständig oder teilweise ungefärbten Bereichen, die den durchlaufenden Mikroporen mit Dimensionen bis zu ca. 20 $\mu$m zugeordnet werden können. Die Proteinverteilung war für alle Membranproben eindeutig zu identifizieren. Allerdings wurden sehr große Unterschiede in Bezug auf Proteinmenge (Fluoreszenzintensität, helle Bereiche) und Proteinverteilung in der Porenstruktur (dunkle Bereiche) gefunden. Die Gesamt-Fluoreszenzintensitäten waren deutlich unterschiedlich, für die erfindungsgemäße Membran nach Beispiel 2 mußte sogar eine geringere Verstärkung als für die anderen Membranen gewählt werden:

Membran aus Beispiel 2 > Sartobind® S Membran >> Membran aus Beispiel 1

Diese Ergebnisse korrelieren gut mit den Angaben zur Bindungskapazität:

Membran nach Beispiel 1: 0,01 mg/cm$^2$
Membran Sartobind® S: 0,90 mg/cm$^2$
Membran nach Beispiel 2: 2,06 mg/cm$^2$

Mit der Untersuchungsmethode konnten eindeutige und große Unterschiede in Bezug auf die Proteinbindung zwischen der etablierten Sartobind® S Membran (Figur 2) sowie den aus dem Beispiel 1 (Figur 3) und dem Beispiel 2 (Figur 4) mit Sulfonsäureliganden funktionalisierten Membranen identifiziert werden. Bei den Membranen aus den Beispielen 1 und 2 korrelieren die Fluoreszenzintensitäten für das Protein (helle Bereiche) mit den nominalen Proteinbindungskapazitäten, d.h. die Membran aus dem Beispiel 1 weist eine nur sehr geringe Bindungskapazität auf, wobei die erfindungsgemäße Membran aus dem Beispiel 2 eine deutlich höhere Bindungskapazität aufweist.

Ausgehend von derselben Porenstruktur des Basisträgers bindet das Protein in den Sartobind® S Membranen vor allem im Volumen der Mikroporen, wobei wesentlich für die Proteinbindung eine dreidimensionale funktionelle Schicht ist. Dabei zeigen diese Membranen an den Rändern der Poren scharfe Abgrenzungen zwischen dem Material der Membran (dunkle Bereiche) und der Proteinschicht (helle Bereiche). Wegen der begrenzten Reichweite dieser funktionellen Schicht verbleiben kleine Anteile am Porenvolumen, in denen kein Protein gebunden ist. Bei der Membran aus dem Beispiel 1 findet die Bindung direkt am Membranmaterial statt, was durch kleine helle Punkte in der Figur 2 zu erkennen ist. Im Gegensatz dazu werden bei der Membran aus dem Beispiel 2 offensichtlich sehr große Mengen an Protein in den Ultraporen der groben Faserstruktur sowie in dem feiner verteilten, faser- oder clusterartigen Membranmaterial gebunden. Zwischen den Verteilungen von Cellulose und Protein wird eine sehr gute Korrelation gefunden, visuell auch daran erkennbar, daß in der Überlagerung nur die Mischfarbe der verwendeten Farbstoffe zu erkennen ist, weil in der Tiefe der Ultraporen sowohl Porenoberfläche als auch Protein sichtbar sind. Der weitaus größte Anteil des Volumens der Mikroporen enthält kein Protein.

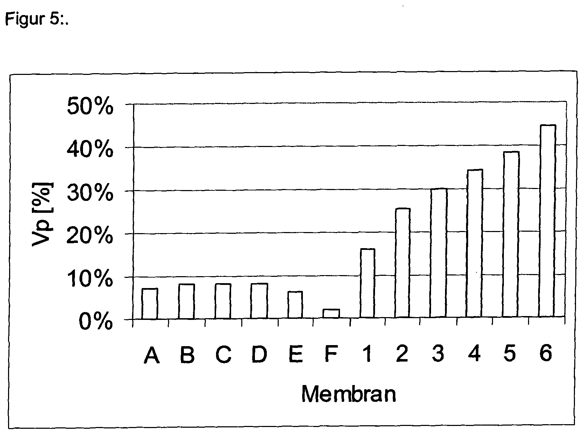

Um die Ultraporen der erfindungsgemäß verwendeten Membranen quantitativ zu erfassen, wurde ein Versuch durchgeführt, in dem die Porenzugänglichkeit für Dextranblau bestimmt wurde. Die Versuchsdurchführung erfolgte in der in Beispiel 14 beschriebenen Weise. Das Ergebnis der Auswertung ist in Tabelle 1 und in Figur 5 gezeigt.

In der Figur 5 ist ein deutlicher Unterschied zwischen den für Dextranblau unzugänglichen Ultraporen für die im Stand der Technik bekannten Membranen A-F und Membranen aus dem Beispiel 14 zu erkennen. Im Falle der Membranen, die unter quellenden Bedingungen verseift wurden, liegt mehr als 15 % des gesamten Porenvolumens im Bereich von Ultraporen (d.h. Poren mit einem Durchmesser <100 nm, die für Dextranblau nicht zugänglich sind), wohingegen es bei den Vergleichsmembranen A-F weniger als 8 % sind.

Demgemäß weisen erfindungsgemäß verwendete Membranen ein Volumen an Ultraporen, die zwar für Wasser zugänglich sind, jedoch nicht für Dextranblau mit einem Molekulargewicht Mw von 2.000.000, von mehr als 15%, vorzugsweise mehr als 18 %, mehr bevorzugt von mehr als 20 %, noch mehr bevorzugt von mehr als 25 % und am meisten bevorzugt von mehr als 30 % des gesamten Porenvolumens auf.

**Figuren**

Figur 1a): Schematische Darstellung der Proteinbindung an Mikroporen einer wie im Beispiel 1 hergestellten und im Stand der Technik bekannten adsorptiven Membran.

Figur 1b): Schematische Darstellung der Proteinbindung an einer adsorptiv wirksamen Polymerbeschichtung von wie in dem Stand der Technik beschriebenen adsorptiven Membranen, die aus einer oder mehreren Trägerstruktur(en) und einer oder mehreren adsorptiv wirksamen Polymerbeschichtung(en) bestehen.

Figur 1c): Schematische Darstellung der Proteinbindung in den Ultraporen einer erfindungsgemäß verwendeten adsorptiven Membran.

Figur 2: CLSM-Bild der Porenmorphologie und Proteinverteilung an der Oberseite der Sartobind S Membran nach Markierung der Cellulose mit Fluoreszenzfarbstoff und Beladung mit Fluoreszenz-markiertem Lysozym.

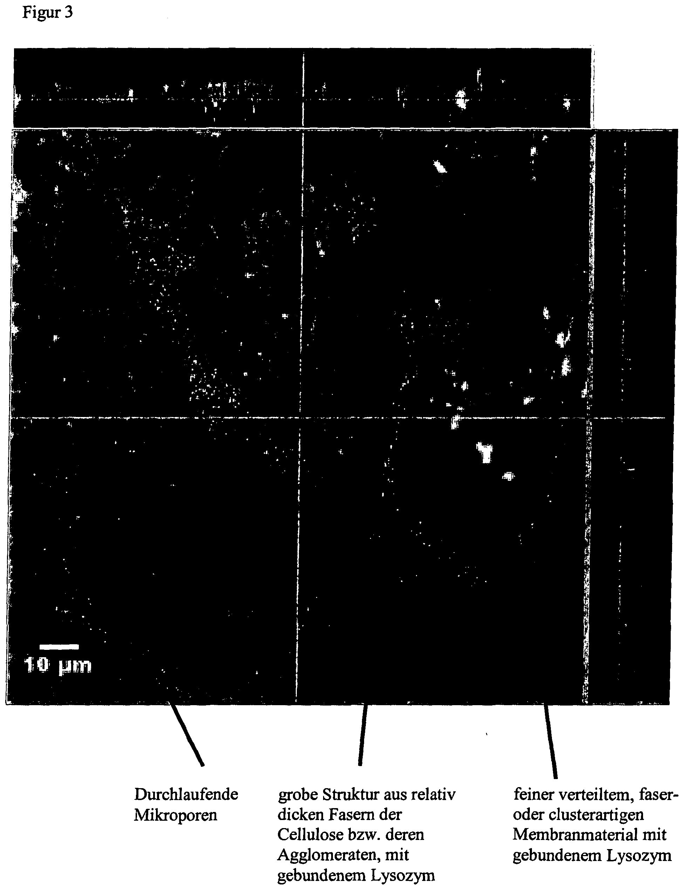

Figur 3: CLSM-Bild der Porenmorphologie und Proteinverteilung an der Oberseite der mit Sulfonsäureliganden umgesetzten Membran nach Beispiel 1 nach Markierung der Cellulose mit Fluoreszenzfarbstoff und Beladung mit Fluoreszenz-markiertem Lysozym.

Figur 4: CLSM-Bild der Porenmorphologie und Proteinverteilung an der Oberseite der mit Sulfonsäureliganden umgesetzten Membran nach Beispiel 2 nach Markierung der Cellulose mit Fluoreszenzfarbstoff und Beladung mit Fluoreszenz-markiertem Lysozym.

Figur 5: Vergleich des prozentualen Anteils von für Dextranblau mit einem Molekulargewicht Mw von 2.000.000 unzugänglichen Poren in den Membranen:

A: Membrane aus Beispiel 1

B-F: Cellulose-Membranen nach dem Stand der Technik 0,2-0,45 μm der Fa. Sartorius Stedim Biotech GmbH 1-6: Membranen aus dem Beispiel 14

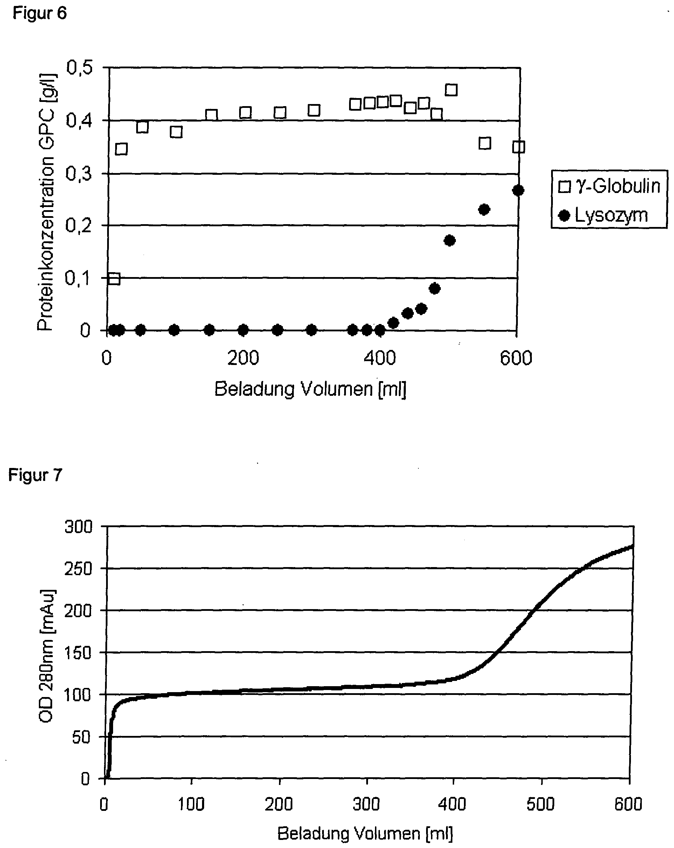

Figur 6: Durchbruchskurve für Lysozym und γ-Globulin aus Beispiel 15, dargestellt aus den GPC-Analysen der einzelnen Fraktionen.

Figur 7: Durchbruchskurve für Lysozym und γ-Globulin aus Beispiel 15, dargestellt durch Detektion der Extinktion (optische Dichte OD) bei 280 nm.

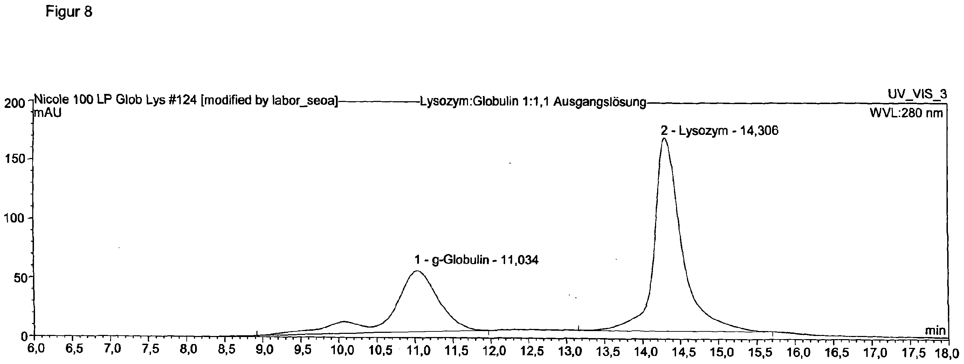

Figur 8: GPC-Analyse der Ausgangslösung aus Beispiel 15.

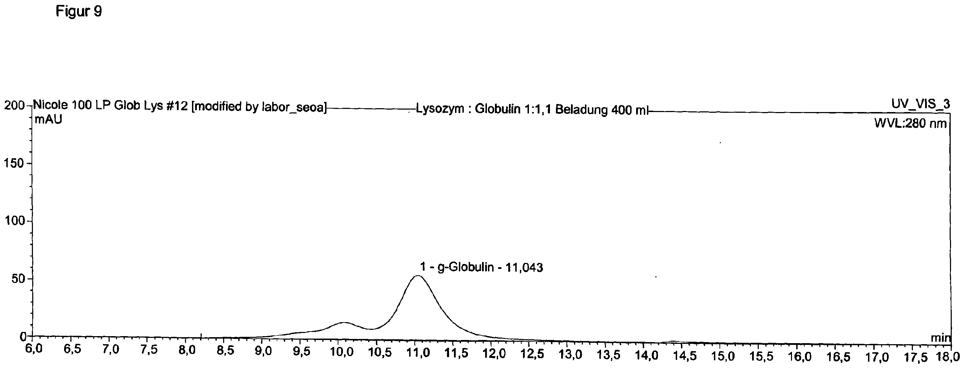

Figur 9: GPC-Analyse der Fraktion des Durchlaufs nach 400 ml Beladung der Membran aus Beispiel 15.

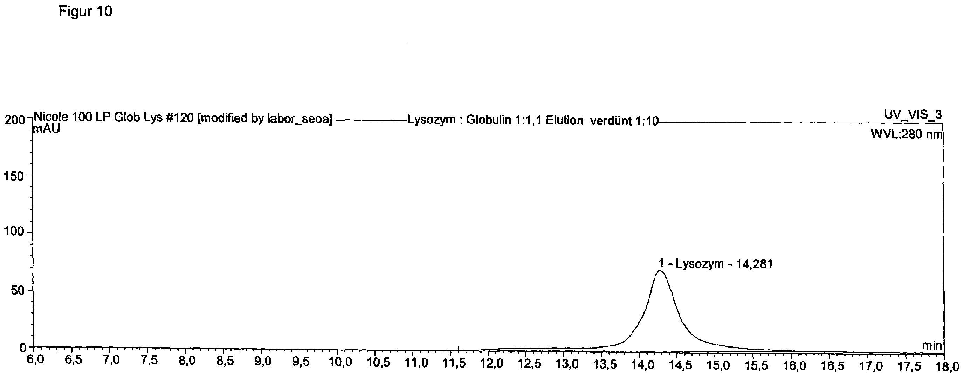

Figur 10: GPC-Analyse der Elutionsfraktion (1:10 verdünnt) aus Beispiel 15.

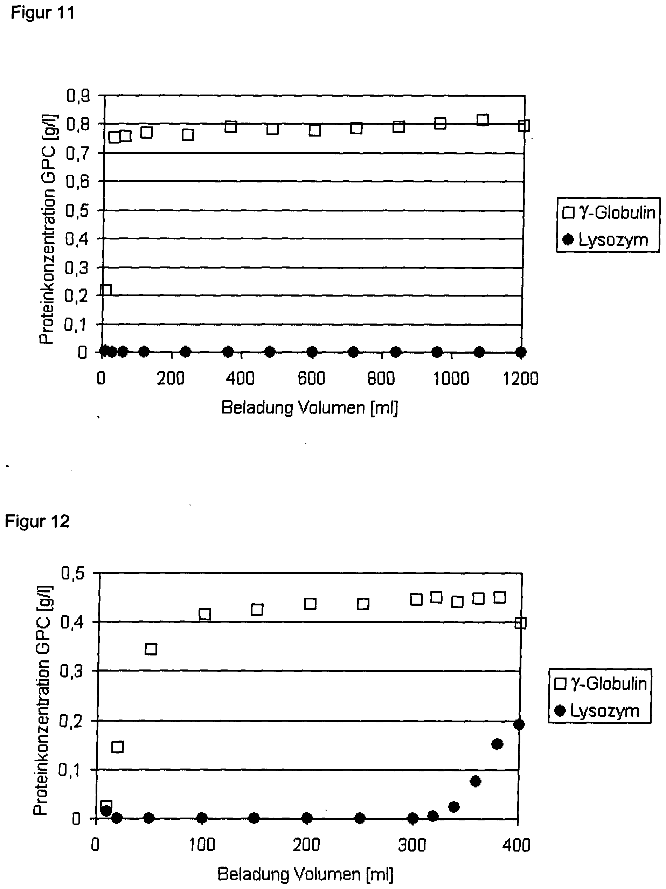

Figur 11: Durchbruchskurve für Lysozym und γ-Globulin aus Beispiel 16, dargestellt aus den GPC-Analysen der einzelnen Fraktionen.

Figur 12. Durchbruchskurve für Lysozym und γ-Globulin aus Beispiel 17, dargestellt aus den GPC-Analysen der einzelnen Fraktionen.

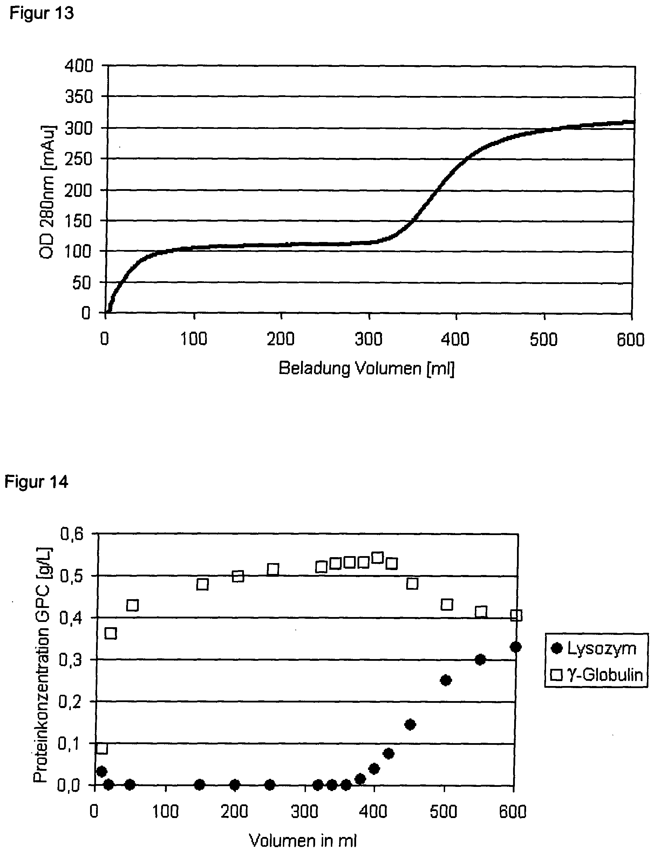

Figur 13: Durchbruchskurve für Lysozym und γ-Globulin aus Beispiel 17, dargestellt durch Detektion der Extinktion (optische Dichte OD) bei 280 nm.

Figur 14: Durchbruchskurve für Lysozym und γ-Globulin aus Beispiel 18, dargestellt aus den GPC-Analysen der einzelnen Fraktionen.

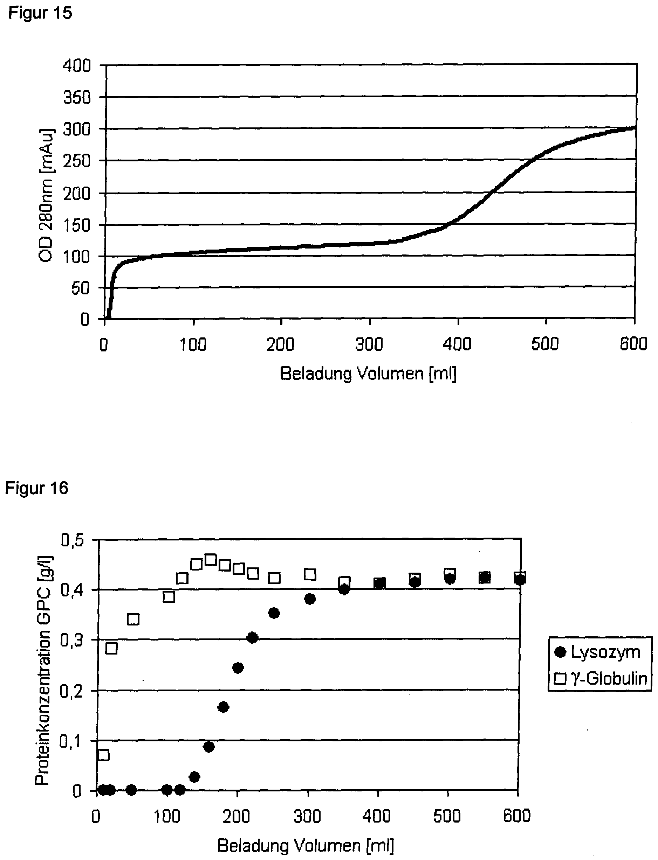

Figur 15: Durchbruchskurve für Lysozum und γ-Globulin aus Beispiel 18, dargestellt durch Detektion der Extinktion (optische Dichte OD) bei 280 nm.

Figur 16: Durchbruchskurve für Lysozym und γ-Globulin aus Vergleichsbeispiel 1, dargestellt aus den GPC-Analysen der einzelnen Fraktionen.

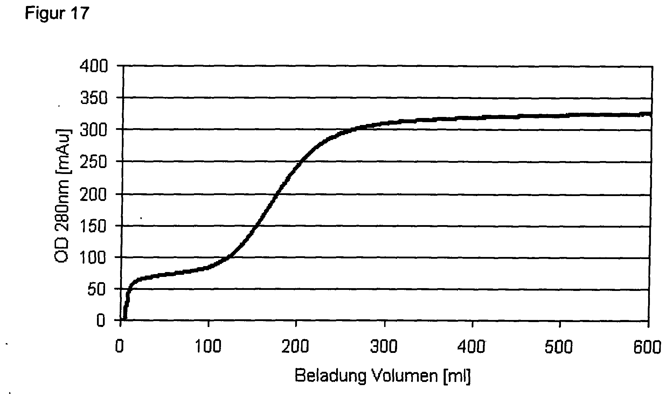

Figur 17: Durchbruchskurve für Lysozym und γ-Globulin aus Vergleichsbeispiel 1, dargestellt durch Detektion der Extinktion (optische Dichte OD) bei 280 nm.

**Beispiele**

Sämtliche Erwähnungen in den Beispielen einer CA-Membran beziehen sich auf einen mit Polyester-Vlies verstärkten Typ einer Celluloseacetat-Membran mit einem Porendurchmesser von ca. 3 μm (gemessen mit einem Coulter-Porometer Typ Capillary Flow Porometer 6.0, CAPWIN Software System, Fa. Porous Materials Inc.), welche einen Wasserdurchfluss von 730 ml/(min x bar x cm$^2$) aufweist. Die Dicke der modifizierten Membranproben betrug durchschnittlich 250 μm. Alle Durchflussangaben sind in ml/(min x bar x cm$^2$)und alle Bindungskapazitätsangaben sind in mg/cm$^2$. Sofern nichts anderes angegeben ist, beziehen sich Prozentangaben auf das Gewicht.

**Beispiel 1**

Aktivierend vernetzte Cellulosehydrat-Membran mit einem niedrigen Quellungsgrad für Vergleichsbeispiele
Die Membran wurde in folgender Weise hergestellt: Als Ausgangsmaterial wurde eine, wie vorstehend genannte CA-Membran verwendet. Diese CA-Membran wurde drei Minuten bei Raumtemperatur mit 15%iger Kalilauge-Lösung in 80%igem Ethanol verseift. Anschließend wurde drei Minuten mit einer 6,8%igen Essigsäurelösung, zweimal mit Ethanol und danach 15 Minuten mit fließendem RO-(d.h. reverse osmosis)-Wasser gespült. Danach wurde die Membran 20 Minuten bei 80 °C im Umlufttrockenschrank getrocknet.
Im nächsten Schritt wurde die so erhaltene, getrocknete Membran 30 Minuten bei Raumtemperatur mit 30%igem 1,4-Butandioldiglycidylether in wässriger 0,1 M Natronlauge-Lösung und wässriger 0,1%iger Natriumborhydrid-Lösung behandelt, und dann die feuchte Membran 20 Stunden in einem abgeschlossenen Gefäß bei Raumtemperatur stehen gelassen.
Abschließend wurde die Membran 30 Minuten mit fließendem Wasser gespült.
Der Wasserdurchfluß der so hergestellten, aktivierend vernetzten Cellulosehydrat-Membran betrug 630 ml/(min x bar x cm$^2$). Der Quellungsgrad betrug 1,16.

**Beispiel 2**

Aktivierend vernetztes Zwischenprodukt für eine erfindungsgemäß verwendete Adsorptionsmembran

Als Ausgangsmaterial wurde eine CA-Membran wie in Beispiel 1 verwendet. Diese CA-Membran wurde 30 Minuten bei Raumtemperatur mit 0,6 M wässriger Natronlauge-Lösung (d.h. unter quellenden Bedingungen) verseift und anschließend 3 x 10 Minuten mit 0,5 M wässriger Natronlauge-Lösung gespült. Die erhaltene Membran wurde 30 Minuten bei Raumtemperatur mit 30%igem 1,4-Butandioldiglycidylether in 0,1 M wässriger Natronlauge-Lösung und 0,1%iger, wässriger Natriumborhydrid-Lösung behandelt (d.h. vernetzt), und danach wurde die feuchte Membran 20 Stunden in einem abgeschlossenen Gefäß bei Raumtemperatur stehengelassen.

Abschließend wurde 30 Minuten mit fließendem Wasser gespült.

Der Wasserdurchfluss des aktivierend vernetzten Zwischenprodukts betrug 45 ml/(min x bar x cm$^2$) und der Quellungsgrad betrug 16,2.

**Beispiel 3**

Aktivierend vernetztes Zwischenprodukt für eine erfindungsgemäß verwendete Adsorptionsmembran: Vorbehandlung der CA-Membran

Eine CA-Membran wurde in gleicher Weise behandelt wie in Beispiel 2, mit der Ausnahme, daß die CA-Membran vor der Verseifung 20 Minuten bei 80 °C im Trockenschrank erhitzt wurde.

Der Wasserdurchfluss des resultierenden, aktivierend vernetzten Zwischenprodukts betrug 21 ml/(min x bar x cm$^2$) und der Quellungsgrad betrug 34,8.

**Beispiel 4**

Aktivierend vernetztes Zwischenprodukt für eine erfindungsgemäß verwendete Adsorptionsmembran: Vorbehandlung der CA-Membran

Eine CA-Membran wurde in gleicher Weise behandelt wie in Beispiel 2 mit der Ausnahme, daß die CA-Membran vor der Verseifung in 20%iger Essigsäure-Lösung auf 80 °C erhitzt wurde und 15 Minuten mit fließendem Wasser gespült wurde.

Der Wasserdurchfluss des resultierenden, aktivierend vernetzten Zwischenprodukts betrug 180 ml/(min x bar x cm$^2$) und der Quellungsgrad betrug 4,06.

**Beispiel 5**

Verschiedene Alkalihydroxide

CA-Membranen wurden jeweils in 0,5 M wässrigen Lösungen von LiOH, NaOH und KOH 30 Minuten bei Raumtemperatur verseift, anschließend ohne Spülung mit wässrigen Lösungen von 15%igem 1,4-Butandioldiglycidylether und 0,1%iger Natriumborhydrid-Lösung in den gleichen Alkalihydroxidlösungen bei Raumtemperatur 3,5 Stunden vernetzt. Die Membranen wurden weiter mit einem quaternären Ammonium-Liganden umgesetzt, indem die vernetzten Membranen 35 Minuten bei 30 °C in einer 10%igen, wässrigen Lösung von Trimethylamin und 5 Minuten bei Raumtemperatur in 5%iger Schwefelsäure-Lösung behandelt und danach 10 Minuten mit fließendem Wasser gespült wurden. Die Ergebnisse sind in der Tabelle 3 angegeben.

**Beispiel 6**

Nichtaktivierend vernetztes Zwischenprodukt für erfindungsgemäß verwendete Adsorptionsmembran

Als Ausgangsmembran wurde eine CA-Membran wie in Beispiel 1 verwendet. Diese CA-Membran wurde 30 Minuten bei Raumtemperatur mit 0,6 M wässriger Natronlauge-Lösung verseift und anschließend 3 x 10 Minuten mit 0,5 M wässriger Natronlauge-Lösung gespült. Die erhaltene Membran wurde 30 Minuten bei Raumtemperatur mit wässrigem, 15%igen 1,4-Butandioldiglycidylether in 0,5 M wässriger Natronlauge-Lösung und 0,1%iger, wässriger Natriumborhydridlösung behandelt (vernetzt) und danach wurde die feuchte Membran 20 Stunden in einem abgeschlossenen Gefäß bei Raumtemperatur stehengelassen. Abschließend wurde 30 Minuten mit fließendem Wasser gespült.

Der Wasserdurchfluss des nicht-aktivierend vernetzten Zwischenprodukts betrug 31 ml/(min x bar x cm$^2$) und der Quellungsgrad betrug 23,5.

**Beispiel 7**

Einführung von quaternären Ammonium-Liganden (Q-Membran)

Aktivierend vernetzte Membranen (Zwischenprodukte) wurden 35 Minuten bei 30 °C in einer 10%igen, wässrigen Lösung von Trimethylamin und 5 Minuten bei Raumtemperatur in 5%iger Schwefelsäure-Lösung behandelt und danach 10 Minuten mit fließendem Wasser gespült, wodurch Membranen mit quaternären Ammonium-Liganden (im folgenden: Q-Membranen) erhalten wurden.

**Beispiel 8**

Einführung von Sulfonsäure-Liganden (S-Membran)

Aktivierend vernetzte Membranen (Zwischenprodukte) wurden 45 Minuten bei 80 °C in einer wässrigen Lösung von 30 % Natriumsulfit und 2,5 % $Na_2HPO_4$ x $H_2O$ bei einem pH-Wert von 8,0 behandelt und danach 10 Minuten mit fließendem Wasser, 5 Minuten mit 35 g einer 1%igen HCl-Lösung, 2 x 5 min mit je 35 g einer wässrigen 1M NaCl-Lösung, 5 min mit 500 g 5%iger $H_2SO_4$-Lösung und 10 Minuten mit fließendem Wasser gespült, wodurch Membranen mit Sulfonsäure-Liganden (S-Membranen) erhalten wurden.

**Beispiel 9**

Einführung von Iminodiessigsäure-Liganden (IDA-Membran)

Aktivierend vernetzte Membranen (Zwischenprodukte) wurden 45 Minuten bei 80 °C mit einer 13%igen, wässrigen Lösung von Iminodiessigsäure bei einem pH-Wert von 11,2 behandelt und danach 10 Minuten mit fließendem Wasser, 5 Minuten mit 1%iger HCl-Lösung, 2 x 5 min mit wässriger 1 M NaCl-Lösung und 10 Minuten mit fließendem Wasser gespült, wodurch Membranen mit Iminodiessigsäure-Liganden (IDA-Membranen) erhalten wurden.

**Beispiel 10**

Einführung von Phenyl-Liganden (Ph-Membran)

Aktivierend vernetzte Membranen (Zwischenprodukte) wurden drei Stunden bei Raumtemperatur mit einer wässrigen Lösung von 1% Anilin in 0,1 M Kpi (Kaliumphosphat)-Puffer bei einem pH-Wert von 8,0 behandelt und die feuchten Proben wurden 19 Stunden im verschlossenen Gefäß belassen. Nach 15 Minuten Spülen mit fließendem Wasser wurde 15 Minuten mit 1 M wässriger NaCl-Lösung und 15 Minuten mit fließendem Wasser nachgespült, wodurch Membranen mit Phenyl-Liganden (Ph-Membranen) erhalten wurden.

**Beispiel 11**

Einführung von p-Aminobenzamidin-Liganden (pABA-Membran)

Eine nicht-aktivierend vernetzte Cellulosehydrat-Membran (Zwischenprodukt) nach Beispiel 6 wurde durch 30minütige Behandlung mit einer 1%igen wässrigen Lösung von Natriumperiodat bei Raumtemperatur aktiviert, 15 Minuten mit fließendem Wasser gespült, eine Stunde bei Raumtemperatur mit einer auf einen pH-Wert von 5,6 eingestellten Lösung von

4,3 g p-Aminobenzamidin-Dihydrochlorid,
2,17g Natrium-Cyanoborhydrid,
2g 1 M Natronlauge, und
34,8g McIlvaine-Puffer mit einem pH-Wert von 5,6 (Gemisch von 0,1 M Citronensäure Monohydrat (Riedel-de-Haen Cat. 33114) und 0,2 M Di-natriumhydrogenphosphat-dihydrat (Merck Cat. 1.06580). 21g Citronensäure Monohydrat in 1l "Reverse-Osmosis"-Wasser (ROW) lösen = 0,1M. 35,6g Di-Natriumhydrogenphosphat-dihydrat in 1l ROW lösen = 0,2M. 500g 0,2 M Di-Natriumhydrogenphosphat-dihydrat vorlegen und mit 0,1 M Citronsäure Monohydrat pH-Wert auf 5,6 einstellen)

behandelt, 15 Minuten mit fließendem Wasser gespült, nacheinander mit 100 g 1%iger wässriger $NaBH_4$-Lösung und 100 g 1 M wässriger NaCl-Lösung behandelt und nochmals 15 Minuten mit fließendem Wasser gespült. Dadurch wurden Membranen mit p-Aminobenzamidin-Liganden (pABA-Membranen) erhalten.

**Beispiel 12**

Einführung von Cibacronblau-3GA-Liganden (CB Membran)

Eine nicht-aktivierend vernetzte Cellulosehydrat-Membran (Zwischenprodukt) nach Beispiel 6 wurde 24 Stunden bei Raumtemperatur mit einer Lösung, hergestellt durch Versetzen einer 10 Minuten bei 80 °C gerührten und bei Raumtemperatur mit 3%iger wässriger Natronlauge-Lösung versetzten 2%igen wässrigen Lösung des Farbstoffs Cibacronblau-3GA behandelt und nacheinander 60 Minuten mit fließendem Wasser, viermal je dreißig Minuten mit Wasser bei 80 °C und 15 Minuten mit fließendem Wasser gespült. Dadurch wurden Membranen mit Cibacronblau-3GA-Liganden (CB-Membranen) erhalten.

**Beispiel 13**

Trocknung der erfindungsgemäß verwendeten Adsorptionsmembran

CA-Membranen wurden in 0,5 M wässriger Natronlauge-Lösung 30 Minuten bei Raumtemperatur verseift, anschließend ohne Spülung mit einer Lösung von 30%igem 1,4-Butandioldiglycidylether und 0,1%igem Natriumborhydrid in 0,5 M wässriger Natronlauge-Lösung bei Raumtemperatur 2,5 Stunden vernetzt, danach mit Trimethylamin derivatisiert und sowohl im ungetrockneten Zustand als auch im bei 80 °C im Umlufttrockenschrank getrockneten Zustand hinsichtlich ihrer statischen Bindungskapazität untersucht. Die Ergebnisse sind in der Tabelle 3 angegeben.

**Beispiel 14**

Bestimmung des Anteils an Ultraporen am Gesamtporenvolumen der Membranen

Für die Bestimmung des Anteils an Ultraporen am Gesamtvolumen der Membranen wurde die CA-Membran bei unterschiedlichen Natronlaugekonzentrationen analog zu Beispiel 2 verseift, mit 0,5M NaOH gespült, wie im Beispiel 2 vernetzt und mit Sulfonsäureliganden wie im Beispiel 8 modifiziert (Membranen 1-6). Zum Vergleich wurden die Membran aus Beispiel 1 (Membran A) und die im Stand der Technik bekannten Mikrofiltrationsmembranen der Fa. Sartorius Stedim Biotech GmbH mit Porengrößen im Bereich 0,2-0,45 $\mu$m (Membranen B-F) verwendet.

Als Dextranblau wurde im Handel erhältliches Dextran aus Leuconostoc mesenteroides, Strain B 512, modifiziert mit Reactive Blue 2 dye, ca. 0,1 mmol Reactive Blue 2 pro Gramm Dextran (Blue Dextran Molecular Weight (Mw) 2.000.000 von Sigma, St. Louis, Missouri, USA, Produktnummer D 5751, CAS-Nummer: 87915-38-6), verwendet.

Der hydrodynamische Durchmesser d dieses Dextranblaus kann mit Hilfe der Gleichung nach Mark-Houwink-Sakurada:

$$d[nm]=0,054 \times Mw^{0,5}$$

berechnet werden und beträgt 76,4 nm.

Ultraporen sind, wie vorstehend definiert, Poren, die für Dextranblau nicht zugänglich sind.

Das für Wasser zugängliche Porenvolumen wird als Vw [$cm^3$] bezeichnet. Es wird angenommen, daß alle Membranporen für Wasser zugänglich sind, so daß Vw dem Gesamtporenvolumen der Membran entspricht.

Das für Dextranblau zugängliche Porenvolumen wird als Vd [$cm^3$] bezeichnet.

Das für Dextranblau nicht zugängliche Porenvolumen der Ultraporen wird als Vp [$cm^3$] bezeichnet.

Vp wird durch das erfindungsgemäße Verfahren, bei dem die Celluloseester-Membran während der Verseifung quillt, vergrößert.

$$\text{Es gilt } Vw = Vd + Vp \text{ und } Vp = Vw - Vd$$

Der prozentuale Anteil von für Dextranblau unzugänglichen Poren in der Membran ist % Vp = 100 x (Vw - Vd)/Vw.

Das für Dextranblau zugängliche Porenvolumen Vd wird nach folgendem Verfahren bestimmt:

10 ml einer Lösung von Dextranblau in RO-Wasser bekannter Konzentration (c0) wird durch eine nasse Membran filtriert. Dadurch wird das Wasser aus dem für Dextranblau zugänglichen Porenvolumen mit der Dextranblau-Lösung ausgetauscht. Die Voraussetzung für die Methode ist, daß die Membran das Dextranblau nicht adsorptiv bindet. Dies ist für unmodifizierte Cellulosehydrat-Membranen, vernetzt und unvernetzt, gegeben. Eine Membran mit einem Durchmesser von 50 mm (d.h. einer Fläche von 19,6 $cm^2$) wird mit fließendem RO-Wasser 15 Minuten intensiv gewaschen. Die nasse Membran wird dann in ein Filtrationsgehäuse eingebaut und 10 ml Dextranblau-Lösung mit einer Konzentration von 5 mg/ml (c0) werden bei 0,1 bar Druck durch die Membran filtriert. Dann wird die Membran aus dem Filtrationsgehäuse ausgebaut, ein Stanzling mit einem Durchmesser von 47 mm (d.h. einer Fläche von

17,3 cm$^2$) wird mittig ausgestanzt (um die abgedichteten Ränder der Membran zu entfernen) und mit einem Labortuch (Kimtech Science, 200, 2, 21 x 20 cm, weiß, 7101) abgetupft.

Danach wird die Membran in einer genau bestimmten Menge (Volumen V = 5,0 ml) RO-Wasser in einem abgedichteten Gefäß 20 Stunden bei 80 Upm geschüttelt. Die Konzentration der Dextranblau-Lösung (c) wird dann photometrisch bei 618 nm bestimmt. Der Extinktionskoeffizient E (1 mg/ml; 1 cm) der Dextranblau-Lösung beträgt 0,896. Aus der Konzentration der Dextranblau-Lösung wird das für Dextranblau zugängliche Porenvolumen berechnet:

$$Vd\ [cm^3] = c \times V/c0$$

Das für Wasser zugängliche Porenvolumen wird nach folgendem Verfahren bestimmt:

Die Membranprobe wird mit fließendem RO-Wasser 15 Minuten intensiv gewaschen. Das an der Membran anhaftende Wasser wird mit dem Labortuch abgetupft und die nasse Membran wird gewogen. Danach wird die Membran bei 80 °C in einem Umlufttrockenschrank 30 Minuten getrocknet und die getrocknete Membran wird gewogen. Die Gewichtsdifferenz zwischen nasser und trockener Membran entspricht der Wassermenge in der Membran (Vw). Dabei wird eine Wasserdichte von 1,0 g/cm$^3$ angenommen.

$$Aus\ \%\ Vp = 100 \times (Vw - Vd)/Vw$$

wird der prozentuale Anteil des für Dextranblau nicht zugänglichen Porenvolumens am Gesamtporenvolumen berechnet.

Mit steigender Natronlaugekonzentration bei der Verseifung der Celluloseacetat-Membran wird die Quellung stärker, der Quellungsgrad steigt, die Permeabilität der Membran sinkt, die Membrandicke nimmt zu, der Anteil an Ultraporen am Gesamtporenvolumen nimmt zu und die Bindungskapazität steigt, wie aus der folgenden Tabelle 1 ersichtlich ist.

Tabelle 1

| | Verseifung | Spülung nach Verseifung | Vp | Permeabilität 10mM Kpi pH7 | Bindungskapazität Lysozym | Quellungsgrad |
|---|---|---|---|---|---|---|
| | c (NaOH) [M] | c(NaOH) [M] | [%] | S Membran [ml/(min x bar x cm$^2$)] | S Membran [mg/cm$^2$] | [-] |
| 1 | 0,20 | 0,5 | 16% | 515 | 0,75 | 1,4 |
| 2 | 0,40 | 0,5 | 25% | 341 | 1,40 | 2,1 |
| 3 | 0,50 | 0,5 | 30% | 180 | 1,71 | 4,1 |
| 4 | 0,60 | 0,5 | 34% | 73 | 1,99 | 10,0 |
| 5 | 0,75 | 0,5 | 39% | 8 | 2,23 | 90,1 |
| 6 | 1,00 | 0,5 | 45% | 4 | 2,40 | 208,6 |

**Beispiel 15**

Trennung von Lysozym und γ-Globulin in Phosphatpuffer mit pH 7,0

Als Modellsystem zur Proteintrennung wurde ein Gemisch aus Lysozym und γ-Globulin benutzt. Einige Parameter der verwendeten Proteine sind wie folgt:

- Lysozym aus Hühnerei der Firma SIGMA, Bestellnummer L-6876/25G, Charge 096K1237 mit einem MG von 14,3 kDa und einem IP von 10,5;
- γ-Globulin aus Rinderserum der Firma SIGMA, Bestellnummer G-5009/100G, Charge 116K7011 mit einem MG von 150 kDa und einem IP von 7-8.

5 Lagen einer S-Membran aus Beispiel 8 wurden in einen Membranhalter eingespannt. Der Membranstapel wies eine Membranfläche von 100 cm$^2$, eine Anströmfläche von 20 cm$^2$ und eine Betthöhe (Dicke des Membranstapels) von 1,4 mm in dem Membranhalter auf. Die Membranen in dem Membranhalter wurden mit 10 mM KPi-Puffer mit einem pH-Wert von 7.0 geflutet, um die Luft zu verdrängen und dann an eine FPLC-Anlage Äkta Explorer 100 der Firma General Electric Health Care angeschlossen.

Bei dem zu trennenden Gemisch handelt es sich um ein Gemisch aus den vorstehend beschriebenen Lysozym und γ-Globulin im Proteinkonzentrationsverhältnis von Lysozym: γ-Globulin von 1,1:1 (0,37 mg/ml Lysozym und 0,33 mg/ml γ-Globulin in 10 mM KPi-Puffer mit einem pH-Wert von 7,0).

Danach wurden die Membranen bzw. der Membranstapel mit einem vier Schritte umfassenden Testprogramm hinsichtlich der Trennung der Proteine untersucht. Die vier Schritte des Testprogramms sind nachfolgend angegeben:

1. Äquilibrieren der Membranen mit 25 ml des 10 mM KPi-Puffers mit einem pH-Wert von 7,0, die mit einer Flußgeschwindigkeit von 5 ml/min durch die Membranen strömen;

2. Beladen der Membran mit 600 ml der Lösung von 0,37 mg/ml Lysozym und 0,33 mg/ml γ-Globulin in 10 mM KPi-Puffer mit einem pH-Wert von 7,0 mit einer Flußgeschwindigkeit von 5 ml/min;

3. Waschen der beladenen Membranen mit 50 ml 10 mM KPi-Puffer mit einem pH-Wert von 7,0 mit einer Flußgeschwindigkeit von 10 ml/min; und

4. Eluieren der beladenen und gewaschenen Membranen mit 50 ml 1 M NaCl in 10 mM KPi-Puffer mit einem pH-Wert von 7,0 mit einer Flußgeschwindigkeit von 10 ml/min.

Die bei der Beladung des Membranstapels durchlaufende Flüssigkeit (Durchlauf) wurde in Fraktionen von jeweils 10 ml gesammelt. Die Konzentrationen von Lysozym und γ-Globulin in den einzelnen Fraktionen wurden mittels GPC bestimmt. Dazu wurde eine HPLC-Anlage der Firma DIONEX mit einer P580 Pumpe, einem ASI-100 Injektor, einem STH 585 Säulenofen und einem UVD 170U UV-Detektor eingesetzt. Als Chromatographiesäule wurde eine Säule PSS Proteema 300 (8x300 mm) der Firma Polymer Standard Sciences verwendet. Die Flußrate betrug 1 ml/min in 50 mM NaPi-Puffer in 0,3 M NaCl mit einem pH-Wert von 6,7 und einer Leitfähigkeit (LF) von 32 mS/cm. Das Injektionsvolumen betrug 0,1 ml und die Ofentemperatur betrug 20 °C. Eine Reinigung der Säule wurde mit 50 mM NaPi-Puffer + 0,05 % Natriumazid mit einem pH-Wert von 6,86 und einer LF von 5,82 mS/cm durchgeführt.

Aus den erhaltenen Chromatogrammen wurden die jeweiligen Mengen an Lysozym und γ-Globulin durch Vergleich der Peakflächen mit den Flächen bekannter Injektionsmengen der Proteine bestimmt.

Die mittels der einzelnen GPC-Analysen ermittelten Mengen an Lysozym bzw. γ-Globulin wurden gegen die jeweilige Fraktionsnummer aufgetragen. Die sich daraus ergebende Durchbruchskurve ist in Figur 6 dargestellt.

In diesem Zusammenhang bedeutet "Durchbruch" einen Anstieg der Proteinkonzentration im Ablauf der Membranadsorbereinheit. Die bis zu diesem Zeitpunkt gebundene Proteinmenge wurde aus der Durchbruchskurve bestimmt.

Weiterhin wurde die Durchbruchskurve durch Detektion der Extinktion, d.h. der optischen Dichte OD bei 280 nm ermittelt. In Figur 7 ist die erhaltene Durchbruchskurve als das UV-Signal in Abhängigkeit von der Beladung dargestellt.

In Figur 8 ist die GPC-Analyse der Ausgangslösung aus Lysozym und γ-Globulin des Beispiels 15, d.h. Lysozym und γ-Globulin im Proteinkonzentrationsverhältnis von 1,1:1, nämlich 0,37 mg/ml Lysozym und 0,33 mg/ml γ-Globulin in 10 mM KPi-Puffer mit einem pH-Wert von 7,0 wiedergegeben.

Aus Figur 9, der GPC-Analyse der Fraktion des Durchlaufes nach 400 ml Beladung, ist ersichtlich, daß das Lysozym aus den ersten 400 ml Lösung vollständig entfernt wurde, wohingegen ein sofortiger Durchbruch von γ-Globulin erfolgte. Der Durchbruch für Lysozym erfolgte erst nach einer Beladung von mehr als 400 ml Lösung (siehe Figur 6). Es wurden 148 mg Lysozym nach einer Beladung von 400 ml Lösung an der Membran gebunden, was einer dynamischen Bindungskapazität bei 0 % Durchbruch von 1,48 mg/cm$^2$ der Membran entspricht.

In Figur 10 ist die GPC-Analyse der Elutionsfraktion (1:10 verdünnt) dargestellt, aus der ersichtlich ist, daß kein γ-Globulin in der Elutionsfraktion gefunden wurde. Zur Elution wurde der Adsorber mit einer Lösung von 1 Mol NaCl im entsprechenden Puffer solange beaufschlagt, bis das UV-Signal des Durchflußphotometers der FPLC-Anlage wieder die Basislinie erreicht hatte. Die gesamte eluierbare Proteinmenge wurde bestimmt.

## Beispiel 16

Trennung von Lysozym und γ-Globulin in Phosphatpuffer mit pH 7,0.

Die Trennung wurde wie in Beispiel 15 durchgeführt mit folgender Änderung: Die Beladung in Schritt 2 erfolgte mit 1200 ml einer Lösung von 0,07 mg/ml Lysozym und 0,77 mg/ml γ-Globulin in 10 mM KPi-Puffer mit einem pH-Wert von 7,0. In Figur 11 sind die mittels der einzelnen GPC-Analysen ermittelten Mengen an Lysozym bzw. γ-Globulin gegen die jeweilige Fraktionsnummer aufgetragen. Das Lysozym wurde mit einer Membranfläche von 100 cm$^2$ vollständig entfernt. Es wurden 84 mg Lysozym nach einer Beladung von 1200 ml an der Membran gebunden. Ähnlich wie in Beispiel 15 wurde auch im Beispiel 16 kein γ-Globulin in der Elutionsfraktion gefunden.

**Beispiel 17**

Trennung von Lysozym und γ-Globulin in Acetatpuffer mit pH 5,0

Die Trennung wurde wie in Beispiel 15 durchgeführt, mit folgender Änderung: Die Beladung in Schritt 2 erfolgte mit 600 ml einer Lösung von 0,37 mg/ml Lysozym und 0,33 mg/ml γ-Globulin in 20 mM Natriumacetat + 50 mM NaCl-Puffer mit einem pH-Wert von 5,0.

In Figur 12 sind die mittels der einzelnen GPC-Analysen ermittelten Mengen an Lysozym bzw. γ-Globulin gegen die jeweilige Fraktionsnummer aufgetragen.

In Figur 13 ist die Durchbruchskurve für Lysozym und γ-Globulin nach Bestimmung der Extinktion (optische Dichte OD) bei 280 nm als das UV-Signal in Abhängigkeit von der Beladung dargestellt.

Es ist ein frühzeitiger Durchbruch von Lysozym bei einem Beladungsvolumen von etwa 350 ml zu beobachten. In der Elutionsfraktion konnte mittels GPC-Analyse auch hier kein γ-Globulin nachgewiesen werden.

**Beispiel 18**

Trennung von Lysozym und γ-Globulin in Phosphatpuffer mit pH 7,0

Die Trennung wurde wie in Beispiel 15 durchgeführt, mit folgender Änderung: Die Beladung in Schritt 2 erfolgte bei einer Flußgeschwindigkeit von 20 ml/min mit 600 ml einer Lösung von 0,43 mg/ml Lysozym und 0,39 mg/ml γ-Globulin in 10 mM KPi-Puffer mit einem pH-Wert von 7,0.

In Figur 14 sind die mittels der einzelnen GPC-Analysen ermittelten Mengen an Lysozym bzw. γ-Globulin gegen die jeweiligen Fraktionsnummern aufgetragen.

In Figur 15 ist die Durchbruchskurve für Lysozym und γ-Globulin nach Bestimmung der Extinktion (optische Dichte OD) bei 280 nm als das UV-Signal in Abhängigkeit von der Beladung dargestellt.

Das Ergebnis hinsichtlich der Trennung ist mit dem aus Beispiel 15 vergleichbar.

**Vergleichsbeispiel 1**

Trennung von Lysozym und γ-Globulin in Phosphatpuffer mit pH 7,0

Die Trennung wurde wie in Beispiel 18 durchgeführt, mit folgender Änderung: Anstelle der S-Membran aus Beispiel 8 wurde eine herkömmliche Sartobind® S Membran der Firma Sartorius Stedim Biotech GmbH verwendet.

In Figur 16 sind die mittels der einzelnen GPC-Analysen ermittelten Mengen an Lysozym bzw. γ-Globulin gegen die jeweiligen Fraktionsnummern aufgetragen.

In Figur 17 ist die Durchbruchskurve für Lysozym und γ-Globulin nach Bestimmung der Extinktion (optische Dichte OD) bei 280 nm als das UV-Signal in Abhängigkeit von der Beladung dargestellt.

Der frühzeitige Durchbruch von Lysozym bei etwa 150 ml Beladung ist deutlich erkennbar und im Gegensatz zu erfindungsgemäß verwendeten Membranen wurde in der Elutionsfraktion γ-Globulin gefunden.

In der folgenden Tabelle 2 sind die Meßwerte für die Proteintrennung von Lysozym und γ-Globulin unter verschiedenen Betriebsbedingungen zusammengefaßt.

Tabelle 2:

| Beispiel | Membran | Puffer | | Ausgangslösung | | Elution | |
|---|---|---|---|---|---|---|---|
| | | | Flussgeschwindigkeit [ml/min] | Lysozym [mg/ml] | $\gamma$-Globulin [mg/ml] | Lysozym [mg/ml] | $\gamma$-Globulin [mg/ml] |
| 15 | Bsp. 8 | KPi pH 7,0 | 5 | 0,37 | 0,33 | 3,10 | n.a[1] |
| 16 | Bsp. 8 | KPi pH 7,0 | 5 | 0,07 | 0,77 | 1,93 | n.a. |
| 17 | Bsp. 8 | Acetat pH 5,0 | 5 | 0,37 | 0,33 | 2,37 | n.a. |
| 18 | Bsp. 8 | KPi pH 7.0 | 20 | 0,43 | 0,39 | 3,29 | n.a. |
| Vergleichsbeispiel 1 | Sartobind® S | KPi pH 7,0 | 20 | 0,43 | 0,39 | 1,76 | 0,127 |
| [1] n.a. bedeutet "nicht nachweisbar" | | | | | | | |

Die Ergebnisse der Bestimmung der statischen Bindungskapazitäten für Lysozym und γ-Globulin und deren Trennung zeigen, daß die erfindungsgemäß verwendeten Membranen deutlich höhere Selektivitäten und verbesserte Trenneigenschaften gegenüber den aus dem Stand der Technik bekannten Membranen aufweisen.

Durch die Kombination der adsorptiven Interaktion des Adsorbenden mit der Membran und des Größenausschlußeffekts der Membran ist es möglich, eine verbesserte Trennung zu erreichen. Die erfindungsgemäß verwendeten Membranen können bei der schnellen Trennung von Biomolekülen unterschiedlicher Größe, aber mit ähnlichen adsorptiven Eigenschaften (z.B. gleich geladene Moleküle auf entgegengesetzt geladener erfindungsgemäßer Membran) eingesetzt werden.

**Auswertung der Membranen**

Die Auswertung der erhaltenen Membranen erfolgte in der nachstehend beschriebenen Weise:

1) Durchflussbestimmung

Membranen mit einer aktiven Membranfläche von 12,5 cm$^2$ wurden jeweils in ein Gehäuse eingebaut und es wurde die Zeit für die Filtration von 100 ml Wasser oder Puffer gemessen. Die in der Tabelle 3 wiedergegebenen Durchflussangaben für mit funktionellen Gruppen umgesetzten Membranen beziehen sich auf den entsprechenden Bindungspuffer. Es wurden die gleichen Puffer verwendet wie für die unten beschriebenen Bestimmungen der Bindungskapazitäten.

2) Bestimmung der statischen Bindungskapazität von Q-Membranen

Membranproben mit einer aktiven Membranfläche von jeweils 17,6 cm$^2$ wurden in 35 ml 20 mM TRIS/HCl pH 7,3 3 x 5 min mit etwa 80 Umdrehungen pro Minute (Upm) geschüttelt. Danach wurden 35 ml einer Lösung von 2 mg/ml Rinderserumalbumin (RSA)-Lösung in 20 mM TRIS/HCl PH 7,3 12-18 Stunden bei 20-25°C mit etwa 80 Upm geschüttelt. Anschließend wurden die Membranproben 2 x 15 Minuten in jeweils 35 ml 20mM TRIS/HCl pH 7,3 gespült. Danach wurden die Membranproben in 20 ml 20 mM TRIS/HCl pH 7,3 + 1 M wässriger NaCl-Lösung geschüttelt. Die Menge des eluierten Proteins wurde durch Messung der optischen Dichte (OD) bei 280 nm bestimmt.

3) Bestimmung der statischen Bindungskapazität von S-Membranen

Membranproben mit einer aktiven Membranfläche von jeweils 17,6 cm$^2$ wurden in 35 ml 10 mM KPi pH 7,0 3 mal 5 Minuten mit etwa 80 Upm geschüttelt. Danach wurden 35 ml einer Lösung von 2 mg/ml Lysozym bzw. 1 mg/ml γ-Globulin in Bindungspuffer 12-18 Stunden bei 20 - 25 °C mit etwa 80 Upm geschüttelt. Anschließend wurden die Membranproben 2 x 15 Minuten in jeweils 35 ml Bindungspuffer gespült. Danach wurden die Membranproben in 20 ml 10 mM KPi pH 7,0 + 1 M wässriger NaCl-Lösung geschüttelt. Die Menge des eluierten Proteins wurde durch Messung der optischen Dichte (OD) bei 280 nm bestimmt.

4) Bestimmung der statischen Bindungskapazität von IDA-Membranen

Membranproben mit einer aktiven Membranfläche von 17,6 cm$^2$ wurden in 35 ml 10 mM KPi pH 7,0 3 x 5 Minuten mit etwa 80 Upm geschüttelt. Danach wurden 35 ml einer Lösung von 2 mg/ml Lysozym in 10 mM KPi pH 7,0 12-18 Stunden bei 20 bis 25°C mit etwa 80 Upm geschüttelt. Anschließend wurden die Membranproben 2 x 15 Minuten in jeweils 35 ml 10 mM KPi pH 7,0 gespült. Danach wurden die Membranproben in 20 ml 10 mM KPi pH 7, 0 + 1 M wässrige NaCl-Lösung geschüttelt. Die Menge des eluierten Proteins wurde durch Messung der optischen Dichte (OD) bei 280 nm bestimmt.

5) Bestimmung der statischen Bindungskapazität von Metallchelat-Membranen (Iminodiessigsäureligand (IDA) komplexiert mit Cu$^{2+}$-Kationen)

IDA-Membranproben mit einer aktiven Membranfläche von 3,1 cm$^2$ wurden in einen Polycarbonatvorsatz eingespannt und an eine Schlauchpumpe angeschlossen. Jeweils 10 ml Lösungen in folgender Reihenfolge wurden mit einer Flußgeschwindigkeit von 2 ml/min mit Hilfe der Schlauchpumpe durch die Membranen gepumpt:

    1. 0,1 M CH$_3$COONa + 0,5 M NaCl pH 4,5
    2. 0,1 M CH$_3$COONa + 0,5 M NaCl pH 4,5 + 0,1 M CuSO$_4$
    3. 0,1 M CH$_3$COONa + 0,5 M NaCl pH 4,5

4. 0,05 M KPi + 0,5 M NaCl pH 7,5

5. 2 mg/ml Cytochrom C in 0,05 M KPi + 0,5 M NaCl pH 7,5

6. 0,05 M KPi + 0,5 M NaCl pH 7,5

7. 0,1 M Imidazol in 0,05 M KPi + 0,5 M NaCl pH 7,5

8. 1 M $H_2SO_4$

Die Menge des eluierten Proteins in Schritt 7 wurde durch Messung der optischen Dichte (OD) bei 528 nm bestimmt.

6) Bestimmung der statischen Bindungskapazität von Ph-Membranen

Membranproben mit einer aktiven Membranfläche von 3,1 cm$^2$ wurden in einen Polycarbonatvorsatz eingespannt und an eine Schlauchpumpe angeschlossen. Lösungen in folgender Reihenfolge wurden mit einer Flußgeschwindigkeit von 2 ml/min mit Hilfe der Schlauchpumpe durch die Membranen gepumpt:

1. 10 ml 0,05 M KPi + 1M $(NH_4)_2$ $SO_4$ NaCl pH 7,0
2. 20 ml 1 mg/ml gamma-Globulin in 0,05 M KPi + 1M $(NH_4)_2SO_4$ NaCl pH 7,0
3. 20 ml 0,05 M KPi + 1M $(NH_4)_2$ $SO_4$ NaCl pH 7,0
4. 10 ml 0,05 M KPi pH 7,0

Die Menge des eluierten Proteins in Schritt 4 wurde durch Messung der optischen Dichte (OD) bei 280 nm bestimmt.

7) Bestimmung der statischen Bindungskapazität von pABA Membranen

Membranproben mit einer aktiven Membranfläche von 3,1 cm$^2$ wurden in einen Polycarbonatvorsatz eingespannt und an eine Schlauchpumpe angeschlossen. Lösungen in folgender Reihenfolge wurden mit einer Flußgeschwindigkeit von 2 ml/min mit Hilfe der Schlauchpumpe durch die Membranen gepumpt:

1. 10 ml 50 mM TRIS/HCl pH 8,8 + 10 mM $CaCl_2$ + 250 mM NaCl
2. 10 ml 2 mg/ml Trypsin Type I in 50 mM TRIS/HCl pH 8,8 + 10 mM $CaCl_2$ + 250 mM NaCl
3. 10 ml 50 mM TRIS/HCl pH 8,8 + 10 mM $CaCl_2$ + 250 mM NaCl
4. 10 ml 0,1 M Glycin/HCl pH 2,8

Die Menge des eluierten Trypsins im Schritt 4 wurde durch Trypsinbestimmung nach Bergmeyer gemäß folgendem Verfahren bestimmt. Die enzymatische Aktivität von Trypsin wird durch Verschiebung der Extinktion von N-$\alpha$-Benzoyl-L-argininethylesterhydrochlorid (BAEE) bei einer Wellenlänge von 253 nm bei der durch Trypsin katalysierten Hydrolyse als $\Delta E$/min bestimmt.
In einer Halbmicroquarzküvette werden die folgenden Lösungen in der angegebenen Reihenfolge gemischt:

1. 850 $\mu$l Puffer (50 mM TRIS/HCl pH8,8 + 10mM $CaCl_2$ + 250mM NaCl),
2. 100 $\mu$l BAEE-Lösung in Bindungspuffer (Sigma Best.Nr. B 4500) und
3. 50 $\mu$l Probe.

Die gefüllte Küvette wird im Photometer platziert und nach 5 s wird $\Delta E$/min bei 253 nm bestimmt.

8) Bestimmung der statischen Bindungskapazität von CB-Membranen

Membranproben mit einer aktiven Membranfläche von 9,8 cm$^2$ wurden in 10 ml 0,1 M wässriger Natronlauge-Lösung 10 Minuten geschüttelt und dann in 10 ml 10 mM KPi pH 7,3 3 x 10 Minuten mit etwa 80 Upm geschüttelt. Danach wurden 5 ml einer Lösung von 2 mg/ml Rinderserumalbumin (RSA) in 10 mM KPi pH 7,0 12-18 Stunden bei 20 - 35°C mit etwa 80 Upm geschüttelt. Anschließend wurden die Membranproben 3 x 10 Minuten in jeweils 10 ml 10 mM KPi pH 7,0 gespült. Danach wurden die Membranproben eine Stunde in 5 ml 10 mM KPi pH 7,0 + 1 M wässriger NaCl-Lösung geschüttelt. Die Menge des eluierten Proteins wurde durch Messung der optischen Dichte (OD) bei 280 mm bestimmt.

9) Konfokale Laserfluoreszenzmikroskopie

Markierung der Membranen

Eine Adsorptionsmembran aus Beispiel 1 und eine Adsorptionsmembran nach Beispiel 2 wurden nach Beispiel 8 mit Sulfonsäureliganden versehen. Zusammen mit einer unter der Bezeichnung Sartobind® S im Handel erhältlichen Adsorptionsmembran der Sartorius Stedim Biotech GmbH mit einem mit Sulfonsäure-belegten Hilfspolymer wurden die Membranen mit dem OH-reaktiven Fluoreszenzfarbstoff "5-DTAF" (5-(4,6-Dichlorotriazinyl)aminofluorescein, Anregungs- bzw. Emissionswellenlänge von 492 nm bzw. 516 nm, Firma Invitrogen) markiert. Die Inkubation der Lösung des Farbstoffs sowie alle folgenden Waschschritte wurden mit jeweils drei Membranproben (Durchmesser: 13 mm) in einem Filterhalter bei kontinuierlicher Durchspülung mit einem Durchfluß von ca. 1 ml/min durchgeführt. Dabei wurden jeweils 20 ml einer 5-DTAF-Lösung in 100 mM Natriumhydrogencarbonat-Lösung mit einem pH-Wert von 9,3 mit an die Membran angepaßten Konzentrationen, nämlich 13,5 $\mu$g/ml 5-DTAF + 100 mM NaCl-Lösung für die Membranen nach den Beispielen 1 und 2 und 25 $\mu$g/ml 5-DTAF + 200 mM NaCl-Lösung für die Sartobind® S Membran, verwendet. Da vermutet wurde, daß die dreidimensionale Kationenaustauscherschicht eine besonders effektive Abschirmung der Cellulosematrix bewirkt, wurde für die Sartobind® S Membran sowohl die Farbstoffals auch die Salzkonzentration erhöht. Nach Durchspülung für ca. 18 Stunden wurden die Proben anschließend nacheinander mit jeweils 100 ml einer 20%igen Ethanollösung, 1M NaCl-Lösung und 200 mM Natriumphosphat-Puffer, pH 7,0, gewaschen. Für die CLSM-Analyse wurde wegen der besten Homogenität der Markierung jeweils die zweite Probe im Filterhalter benutzt.

Markierung und Reinigung des Proteins

Lysozym (erhältlich von der Firma Sigma, St. Louis, Missouri, USA; Protein ca. 95%, ca. 50 000 units/mg Protein) wurde mit dem $NH_2$-reaktiven Fluoreszenzfarbstoff "Cy5 mono-reactive NHS Ester" (erhältlich von der Firma GE Health Care Bio-Sciences AB, Uppsala, Schweden) in Natriumcarbonat-Puffer, pH 9,3, markiert sowie anschließend zunächst durch Gelfiltration und dann durch HPIonenaustauschchromatographie gereinigt. Durch entsprechende Auswahl der Fraktionen der Chromatographie wurde das einfach markierte Lysozym in reiner Form erhalten. Danach erfolgte mittels Ultrafiltration die Aufkonzentrierung auf die für das Bindungsexperiment notwendige Konzentration. Die Bestimmung der Konzentration des markierten Lysozyms erfolgte mittels UV-Vis-Photometer (Messung der Absorptionen bei 280 nm und 650 nm).

Inkubation der Membranen mit Protein

Proben der mit 5-DTAF-markierten Membranen wurden mit einem Durchmesser von 5 mm ausgestanzt und für vier Stunden in einer Lösung des markierten Lysozyms mit einer Konzentration von 0,6 g/L in 200 mM Natriumphosphat-Puffer, pH 7,0 + 50 mM wässrige NaCl-Lösung inkubiert (für 1 cm$^2$ Proben wurden jeweils 4,1 ml Proteinlösung eingesetzt). Danach wurden die Proben mit dem Puffer für 15 Minuten gewaschen.

CLSM Analyse

Die Analyse erfolgte mit dem CLSM-System Leica TCS SP. Jede Probe wurde in 200 mM Natriumphosphat-Puffer von beiden Oberflächen aus untersucht. Zunächst erfolgte die Bestimmung der geeigneten Signalverstärkung (Kriterien: unterdrückte Autofluoreszenz der Membran; das Maximum der Signalverstärkung wurde mit Hilfe des Histogramms in der Auswertesoftware "Zeiss LSM Image Browser" eingestellt, um örtliche Überbelichtungen zu vermeiden) und Identifizierung von z = 0 (Kriterien: hohe Streuintensität und anschließende erstmalige Identifizierung der Porenmorphologie bei weiterer Verringerung des Abstands zur Probe). Danach wurde in x,y-Scans an verschiedenen z-Positionen die aus SEM bekannte charakteristische Morphologie der Sartobind® S Membranen gesucht. Danach erfolgten in einem engen Bereich verschiedener z-Positionen (in ca. 20 Mikrometer Tiefe, in Abständen von 1 Mikrometer in beide Richtungen) detaillierte x, y-Scans der beiden Anregungswellenlängen (488 nm für 5-DTAF, 633 nm für Cy5). Für jede Probe und jede Orientierung wurden diese Scans jeweils für drei verschiedene Positionen durchgeführt. Die Probe Sartobind® S wurde zuerst vermessen; die für diese Probe gewählten Einstellungen (z-Position und Signalverstärkung) wurden bei der Messung der anderen Membranproben beibehalten. Weil die Signalintensitäten von der Membran nach Beispiel 2 bei 633 nm sehr viel höher als bei den anderen beiden Membranen waren, wurde eine Verringerung der Signalverstärkung vorgenommen:

"Gains" (488 nm/633nm)
Membran Sartobind® S: 426/643
Membran nach Beispiel 1: 426/669

Membran nach Beispiel 2: 357/650

CLSM Auswertung

Die Auswertungen erfolgten mit Hilfe des Zeiss LSM Image Browsers 3.5.0.376. Aus den erhaltenen Bildern wurden detaillierte x,y-Scans in einem Bereich der z-Positionen von ca. 20 $\mu$m Tiefe für die Oberseite ausgewählt. Die erhaltenen Bilder wurden jeweils als 8-Bit Bilder mit einer Auflösung von 512 x 512 Pixel, entsprechend 146,2 x 146,2 $\mu$m$^2$, dargestellt. Die Figuren 2 bis 4 zeigen die Überlappung der beiden Bilder der Verteilung von Lysozym und der Porenmorphologie der Cellulose. Zusätzlich wird für jede Messung am oberen und rechten Rand der Darstellung auch noch ein Intensitätsprofil der Intensitäten für beide Fluoreszenzmarkierungen gezeigt.

Ergebnisse der Versuche

Die Ergebnisse der Versuche sind in der nachstehenden Tabelle 3 gezeigt.

Tabelle 3

| Membran aus Beispiel | Bemerkung | Ligand | Protein | Durchfluss | Bindungskapazität |
|---|---|---|---|---|---|
| 1 | | Q | RSA | 643 | 0,07 |
| | | S | Lysozym | 664 | 0,01 |
| | | IDA | Lysozym | 681 | 0,03 |
| | | IDA+Cu2+[1] | Cytochrom C | 681 | 0,15 |
| | | Ph | gamma-Globulin | 570 | 0,2 |
| 2 | | Q | RSA | 44 | 0,92 |
| | | S | Lysozym | 38 | 2,06 |
| | | IDA | Lysozym | 41 | 1,91 |
| | | IDA+Cu2+[1] | Cytochrom C | 41 | 0,5 |
| | | Ph | gamma-Globulin | 31 | 1,26 |
| 3 | | Q | RSA | 20 | 1,13 |
| | | S | Lysozym | 24 | 2,85 |
| 4 | | Q | RSA | 158 | 0,74 |
| | | S | Lysozym | 167 | 3,11 |
| | | S | gamma-Globulin | 167 | 0,44 |
| 5 | LiOH | Q | RSA | 70 | 1,18 |
| | NaOH | Q | RSA | 109 | 0,93 |
| | KOH | Q | RSA | 519 | 0,08 |
| 6 | | CB | RSA | 30 | 0,31 |
| | | pABA | Trypsin | 35 | 0,75 |
| 13 | ungetrocknet | Q | RSA | 213 | 0,94 |
| | getrocknet | Q | RSA | 239 | 0,92 |
| [1] Metallchelat aus Iminodiessigsäureligand (IDA) komplexiert mit Cu$^{2+}$-Kationen | | | | | |

**Vergleichsbeispiel 2**

Gleichzeitige Verseifung und Vernetzung wie im Beispiel 1, Probe K10C der WO 2007/017085 A2, aber mit 1,4-Butandioldiglycidylether anstelle von Epichlorhydrin, unter nicht-quellenden Bedingungen.

Es wurden eine wie vorstehend definierte CA-Membran und eine 0,65 $\mu$m Celluloseacetat-Membran wie in Bsp. 1, Probe K10C der WO 2007/017085 A2 mit einem Wasserdurchfluß von 65 ml/(min x bar x cm$^2$) als Ausgangsmembranen verwendet.

Die Celluloseacetatmembranen wurden in 100 g Wasser, 10 g Na$_2$SO$_4$ und 1 g 1,4-Butandioldiglycidylether auf 47 °C erhitzt und 10 g einer 1M wässrigen Natronlauge-Lösung wurden während 30 Minuten zudosiert. Die Membranen wurden weiter in der Lösung 3,5 Stunden bei 47 °C behandelt und anschließend 30 Minuten mit fließendem Wasser gespült. In die Membranen wurden quaternäre Ammonium-Liganden eingeführt, wobei Q-Membranen erhalten wurden. Die aus der CA-Membran erhaltene, verseifte und vernetzte Q-Membran wies einen Wasserdurchfluss von 589 ml/(min x bar x cm$^2$), einen Quellungsgrad von 1,2 und eine Bindungskapazität für RSA von 0,04 mg/cm$^2$ auf. Die aus der Celluloseacetat-Membran gemäß Beispiel 1, Probe K10C der WO 2007/017085 A2 erhaltene, verseifte und vernetzte Q-Membran wies einen Wasserdurchfluss von 66 ml/(min x bar x cm$^2$), einen Quellungsgrad von 1,0 und eine Bindungskapazität für RSA von 0,04 mg/cm$^2$ auf.

Vergleichsbeispiel 3 Versuch zur Quellung von bereits verseiften Cellulosehydrat-Membranen

Eine als Ausgangsmembran verwendete und wie vorstehend definierte CA-Membran wurde drei Minuten bei Raumtemperatur mit einer 15%igen Kalilauge-Lösung in 80%igem Ethanol verseift und anschließend drei Minuten mit einer 6,8%igen Essigsäure-Lösung, zweimal mit Ethanol und 15 Minuten mit fließendem RO-Wasser gespült. Die erhaltene, verseifte Membran wurde 30 Minuten bei Raumtemperatur mit einer 0,6 M wässrigen Natronlauge behandelt und danach dreimal 10 Minuten mit einer 0,5 M wässrigen Natronlauge gespült. Anschließend wurde die Membran 30 Minuten bei Raumtemperatur mit einer 30%igen Lösung von 1,4-Butandioldiglycidylether in 0,1 M wässriger Natronlauge-Lösung und 0,1%iger, wässriger Natriumborhydrid-Lösung behandelt, worauf die feuchte Membran 20 Stunden in einem abgeschlossenen Gefäß bei Raumtemperatur stehengelassen wurde. Schließlich wurde die erhaltene Membran 30 Minuten mit fließendem Wasser gespült.

Der Wasserdurchfluss der so hergestellten, verseiften und vernetzten Cellulosehydrat-Membran betrug 688 ml/(min x bar x cm$^2$) und der Quellungsgrad betrug 1,06.

In zwei Proben der Membran wurden, wie in den Beispielen 7 bzw. 8 beschrieben, quaternäre Ammoniumliganden bzw. Sulfonsäureliganden eingeführt, wodurch eine Q-Membran und eine S-Membran erhalten wurden. Die Q-Membran wies eine Bindungskapazität für RSA von 0,044 mg/cm$^2$ auf und die S-Membran eine Bindungskapazität für Lysozym von 0,067 mg/cm$^2$.

## Patentansprüche

1.  Verfahren für die Trennung eines Stoffgemisches aus mindestens zwei Komponenten unterschiedlichen mittleren Molekulargewichts Mw in einem Bereich von 4.000 bis 4.000.000, umfassend die Schritte:

    A) Kontaktieren des Stoffgemisches in einem flüssigen Medium mit mindestens einem flächigen Adsorbens und adsorptives Abreichern bevorzugt der mindestens einen Komponente mit niedrigerem mittleren Molekulargewicht aus dem flüssigen Medium durch das mindestens eine flächige Adsorbens und
    B) Trennen des mindestens einen flächigen Adsorbens aus Schritt A) von dem flüssigen Medium, welches die mindestens eine Komponente mit höherem mittleren Molekulargewicht enthält,
    wobei das mindestens eine flächige Adsorbens eine vernetzte Cellulosehydrat-Membran mit einer porösen Doppelstruktur ist, welche besteht aus:

    - Mikroporen mit einem Durchmesser im Bereich von >100 nm bis 20 μm und
    - Ultraporen mit einem Durchmesser von < 100 nm, die für Dextranblau mit einem mittleren Molekulargewicht Mw von 2.000.000 nicht zugänglich sind und wobei der Anteil des Volumens der Ultraporen an dem für Wasser zugänglichen Gesamtporenvolumen mehr als 15% beträgt, erhältlich durch Verseifen einer Celluloseester-Membran mit einem Porendurchmesser von 0,1 bis 20 μm unter quellenden Bedingungen in einem Natriumhydroxid oder Lithiumhydroxid enthaltenden wässrigen Verseifungsmedium, wobei die Konzentration des Natriumhydroxids oder Lithiumhydroxids im Verseifungsmedium 0,4 Gew.-% bis 50 Gew.-%, bezogen auf das Verseifungsmedium, beträgt, und nachfolgendes Vernetzen der verseiften Membran mit mindestens einem Vernetzungsmittel, welches bzw. welche mindestens zwei funktionelle Gruppen im Molekül aufweist bzw. aufweisen, die mit den Hydroxylgruppen der Cellulose reaktiv sind,

    wobei mindestens eine funktionelle Gruppe an die Membran gebunden ist, wobei die funktionellen Gruppen Liganden sind, die befähigt sind, mit in Medien enthaltenen Adsorbenden Wechselwirkungen einzugehen.

2.  Verfahren nach Anspruch 1, wobei nach Schritt B) die Schritte folgen:

    C) Kontaktieren des flächigen Adsorbens aus Schritt B) mit mindestens einem zweiten flüssigen Medium unter Desorbieren mindestens einer Komponente des Stoffgemisches aus mindestens zwei Komponenten und
    D) Trennen des mindestens einen flächigen Adsorbens aus Schritt C) von dem flüssigen Medium aus Schritt C).

3. Verfahren nach Anspruch 1 oder 2, wobei die Liganden anionische und/oder kationische Gruppen umfassen.

4. Verfahren nach Anspruch 3, wobei die kationischen Gruppen ausgewählt sind aus der Gruppe der primären, sekundären, tertiären und/oder quaternären Amine.

5. Verfahren nach Anspruch 3, wobei die anionischen Gruppen ausgewählt sind aus der Gruppe der Sulfonsäuren, Phosphorsäuren und Carbonsäuren.

6. Verfahren nach Anspruch 1 oder 2, wobei die Liganden Affinitätsliganden sind.

7. Verfahren nach Anspruch 6, wobei die Affinitätsliganden ausgewählt sind aus der Gruppe, umfassend Benzamidine, biomimetische Liganden und/oder Proteine.

8. Verfahren nach Anspruch 1 oder 2, wobei die Liganden ausgewählt sind aus der Gruppe der Metallchelate.

9. Verfahren nach Anspruch 1 oder 2, wobei die Liganden ausgewählt sind aus der Gruppe der hydrophoben Liganden.

10. Verfahren nach Anspruch 9, wobei die Liganden ausgewählt sind aus C1-C20-Alkyl und deren Derivaten oder C6-C25-Aryl und deren Derivaten oder C7-C25-Arylalkyl und deren Derivaten oder -[(CH2)m-O-]n-R, wobei m 2 oder 3 ist, n eine ganze Zahl von größer gleich 1 ist und wobei R -H oder -C1-C5-Alkyl bedeuten.

11. Verfahren nach Anspruch 1 oder 2, wobei die Liganden ausgewählt sind aus der Gruppe der reaktiven Epoxid-, Aldehyd-, Azlacton-, N-Hydroxysuccinimid- und/oder Carbodiimid-Gruppen.

12. Verfahren nach Anspruch 1 oder 2, wobei die Komponente mit dem höheren mittleren Molekulargewicht ein Antikörper oder dessen Aggregat ist.

13. Verfahren nach Anspruch 1 oder 2, wobei die Komponente mit dem höheren mittleren Molekulargewicht ein Virus ist.

**Claims**

1. A method for separating a mixture of materials comprising at least two components of different average molecular weight Mw in a range from 4000 to 4 000 000, comprising the steps:

A) contacting the mixture of materials in a liquid medium with at least one flat adsorbent and adsorptively depleting preferably the at least one component having a lower average molecular weight from the liquid medium through the at least one flat adsorbent, and
B) separating the at least one flat adsorbent from step A) from the liquid medium, which comprises the at least one component having a higher average molecular weight,
wherein the at least one flat adsorbent is a crosslinked cellulose hydrate membrane having a porous double structure consisting of:

- micropores having a diameter in the range from >100 nm to 20 $\mu$m, and
- ultrapores having a diameter of <100 nm, which are not accessible to Blue Dextran having an average molecular weight Mw of 2 000 000, and

wherein the fraction of the volume of the ultrapores is more than 15% of the entire pore volume accessible to water, obtainable by
hydrolyzing a cellulose ester membrane having a pore diameter of from 0.1 to 20 $\mu$m under swelling conditions in an aqueous hydrolysis medium containing sodium hydroxide or lithium hydroxide, wherein the concentration of the sodium hydroxide or lithium hydroxide in the hydrolysis medium is from 0.4% by weight to 50% by weight, based on the hydrolysis medium, and subsequently, crosslinking the hydrolyzed membrane with at least one crosslinking agent which has at least two functional groups in the molecule which are reactive with the hydroxyl groups of the cellulose,
wherein at least one functional group is bonded to the membrane, wherein the functional groups are ligands which are capable of entering into interactions with adsorbates present in media.

2. The method according to claim 1, wherein the following steps follow step B):

> C) contacting the flat adsorbent from step B) with at least one second liquid medium with desorption of at least one component of the mixture of materials comprising at least two components, and
> D) separating the at least one flat adsorbent from step C) from the liquid medium from step C).

3. The method according to claim 1 or 2, wherein the ligands comprise anionic and/or cationic groups.

4. The method according to claim 3, wherein the cationic groups are selected from the group of the primary, secondary, tertiary, and/or quaternary amines.

5. The method according to claim 3, wherein the anionic groups are selected from the group of the sulfonic acids, phosphoric acids, and carboxylic acids.

6. The method according to claim 1 or 2, wherein the ligands are affinity ligands.

7. The method according to claim 6, wherein the affinity ligands are selected from the group comprising benzamidines, biomimetic ligands, and/or proteins.

8. The method according to claim 1 or 2, wherein the ligands are selected from the group of the metal chelates.

9. The method according to claim 1 or 2, wherein the ligands are selected from the group of the hydrophobic ligands.

10. The method according to claim 9, wherein the ligands are selected from C1-C20-alkyl and their derivatives or C6-C25-aryl and their derivatives or C7-C25-arylalkyl and their derivatives or -[(CH2)m-O-]n-R, where m is 2 or 3, n is an integer greater than or equal to 1, and R is -H or -C1-C5-alkyl.

11. The method according to claim 1 or 2, wherein the ligands are selected from the group of the reactive epoxide, aldehyde, azlactone, N-hydroxysuccinimide, and/or carbodiimide groups.

12. The method according to claim 1 or 2, wherein the component having the higher average molecular weight is an antibody or an aggregate thereof.

13. The method according to claim 1 or 2, wherein the component having the higher average molecular weight is a virus.

**Revendications**

1. Procédé pour la séparation d'un mélange de matières comportant au moins deux composantes présentant des poids moléculaires moyens Mw différents dans une gamme allant de 4000 à 4000000, procédé comportant les étapes suivantes :

> A) la mise en contact du mélange de matières en milieu liquide avec au moins un adsorbant surfacique et appauvrissement par adsorption du milieu liquide préférentiellement en ladite au moins une composante ayant le poids moléculaire moyen plus bas moyennant ledit au moins un adsorbant surfacique, et
> B) séparation dudit au moins un adsorbant surfacique de l'étape A) du milieu liquide qui comporte ladite au moins une composante ayant le poids moléculaire moyen plus élevé,

ledit au moins un adsorbant surfacique étant une membrane d'hydrate de cellulose réticulé présentant une structure double poreuse qui est constituée par :

> - des micropores ayant un diamètre dans la gamme allant de >100 nm à 20 $\mu$m, et
> - des ultra-pores ayant un diamètre de <100 nm qui ne sont pas accessibles au bleu de dextrane ayant un poids moléculaire moyen de 2000000, la fraction volumétrique des ultra-pores par rapport au volume total de pores accessibles à l'eau étant de plus de 15%, obtenable par saponification d'une membrane d'ester de cellulose présentant des diamètres de pores allant de 0,1 à 20 $\mu$m dans des conditions de gonflement en milieu de saponification aqueux comportant de l'hydroxyde de sodium ou de lithium, la concentration en hydroxyde de sodium ou de lithium dans le milieu de saponification étant de 0,4 % en poids à 50 % en poids, par rapport au milieu de saponification, et ensuite réticulation de la membrane saponifiée à l'aide d'au moins un agent de réticulation qui comporte au moins deux groupements fonctionnels dans la molécule, qui sont réactifs avec les groupements hydroxyle de la cellulose, au moins un groupement fonctionnel étant lié à la membrane, les groupements fonctionnels étant des ligands qui sont aptes à interagir avec des adsorbants présents dans des milieux.

2. Procédé selon la revendication 1 dans lequel l'étape B) est suivie par :

C) la mise en contact de l'adsorbant surfacique de l'étape B) avec au moins un deuxième milieu liquide et désorption d'au moins une composante du mélange de matières comportant au moins deux composantes, et
D) la séparation dudit au moins un adsorbant surfacique de l'étape C) du milieu liquide de l'étape C).

3. Procédé selon la revendication 1 ou 2 dans lequel les ligands comportent des groupements anioniques et cationiques.

4. Procédé selon la revendication 3 dans lequel les groupements cationiques sont choisis dans le groupe des amines primaires, secondaires, tertiaires et/ou quaternaires.

5. Procédé selon la revendication 3 dans lequel les groupements anioniques sont choisis dans le groupe des acides sulfoniques, des acides phosphoniques et des acides carboxyliques.

6. Procédé selon la revendication 1 ou 2 dans lequel les ligands sont des ligands d'affinité.

7. Procédé selon la revendication 6 dans lequel les ligands d'affinité sont choisis parmi le groupe comportant des benzamidines, des ligands biomimétiques et/ou des protéines.

8. Procédé selon la revendication 1 ou 2 dans lequel les ligands sont choisis parmi le groupe des chélates de métaux.

9. Procédé selon la revendication 1 ou 2 dans lequel les ligands sont choisis parmi le groupe des ligands hydrophobes.

10. Procédé selon la revendication 9 dans lequel les ligands sont choisis parmi $C_1$-$C_{20}$-alkyle et dérivés de ceux-ci ou $C_6$-$C_{25}$-aryle et dérivés de ceux-ci ou $C_7$-$C_{25}$-arylalkyle et dérivés de ceux-ci ou -[$(CH_2)_m$-O-]$_n$-R, m étant 2 ou 3, n étant un nombre entier plus grand ou égal à 1 et R étant -H ou -$C_1$-$C_5$-alkyle.

11. Procédé selon la revendication 1 ou 2 dans lequel les ligands sont choisis parmi le groupe des groupements réactifs époxyde, aldéhyde, azlactone, N-hydroxysuccinimide et/ou carbodiimide.

12. Procédé selon la revendication 1 ou 2 dans lequel la composante ayant le poids moléculaire moyen plus élevé est un anticorps ou un agrégat de celui-ci.

13. Procédé selon la revendication 1 ou 2 dans lequel la composante ayant le poids moléculaire moyen plus élevé est un virus.

**Figur 1**

a) b) c)

Träger    Adsorbend    Polymerbeschichtung    Ultraporen

Figur 2

An der adsorptiv
wirksamen
Polymerschicht
gebundenes Lysozym

Durchlaufende
Mikroporen

grobe Struktur aus relativ
dicken Fasern der
Cellulose bzw. deren
Agglomeraten, adsorptiv
unwirksam

feiner verteiltem, faser-
oder clusterartigen
Membranmaterial

Figur 3

| Durchlaufende Mikroporen | grobe Struktur aus relativ dicken Fasern der Cellulose bzw. deren Agglomeraten, mit gebundenem Lysozym | feiner verteiltem, faser- oder clusterartigen Membranmaterial mit gebundenem Lysozym |

Figur 4

| Durchlaufende Mikroporen | grobe Struktur aus relativ dicken Fasern der Cellulose bzw. deren Agglomeraten mit für Biomoleküle zugänglichen Ultraporen mit gebundenem Lysozym | feiner verteiltem, faser- oder clusterartigen Membranmaterial mit für Biomoleküle zugänglichen Ultraporen mit gebundenem Lysozym |

Figur 5:.

Figur 6

Figur 7

Figur 8

Figur 9

EP 2 271 419 B1

Figur 10

EP 2 271 419 B1

Figur 11

Figur 12

Figur 13

Figur 14

Figur 15

Figur 16

Figur 17

**EP 2 271 419 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7153426 B2
- WO 03015902 A2
- US 6103121 A
- WO 2007017085 A2
- DE 4323913 A1
- WO 2008095709 A1

**In der Beschreibung aufgeführte Nicht-Patentliteratur**

- **VON DONGMEI ZHOU ; HANFA ZOU ; HAILIN WANG ; JIANYI NI ; QIANG ZHANG ; YUKUI ZHANG.** Alkaline treatment of the cellulose fiber affecting membrane column behaviour for high-performance immunoaffinity chromatography. *Biomed. Chromatogr.,* 2000, vol. 14, 511-515
- *CHEMICAL ABSTRACTS,* 87915-38-6
- **GREG T. HERMANSON ; A. KRISHNA MALLIA ; PAUL K. SMITH.** Immobilized Affinity Ligand Techniques. Academic Press, INC, 1992